# EUROPÄISCHE PATENTSCHRIFT

(11) **EP 1 711 612 B1**
(45) Veröffentlichungstag und Bekanntmachung des Hinweises auf die Patenterteilung: **25.07.2007**
(21) Anmeldenummer: 04740603.8
(22) Anmeldetag: 03.07.2004
(51) Int. Cl.: C12N 15/82

(54) **EXPRESSIONSKASSETTEN ZUR BIDIREKTIONALEN TRANSGENEN EXPRESSION VON NUKLEINSÄUREN IN PFLANZEN**
EXPRESSION CASSETTES FOR THE BI-DIRECTIONAL TRANSGENIC EXPRESSION OF NUCLEIC ACIDS IN PLANTS
CASSETTES D'EXPRESSION PERMETTANT L'EXPRESSION TRANSGENIQUE BIDIRECTIONNELLE D'ACIDES NUCLEIQUES CHEZ DES VEGETAUX

(30) Priorität: 22.07.2003 DE 10333479
(43) Veröffentlichungstag der Anmeldung: 18.10.2006
(73) Patentinhaber: SunGene GmbH, 06466 Gatersleben (DE)
(72) Erfinder: HEIM, Ute, 06466 Gatersleben (DE); HERBERS, Karin, 06484 Quedlinburg (DE); KUNZE, Irene, 06466 Gatersleben (DE)
(74) Vertreter: Pressler, Uwe
(86) Internationale Anmeldenummer: PCT/EP2004/007255
(87) Internationale Veröffentlichungsnummer: WO 2005/019459

(56) Entgegenhaltungen:
- WO-A-03/006660
- US-A1- 2002 108 142
- DATABASE EMBL 8. Juli 2002 (2002-07-08), "Sequence 315 from patent WO0198480." XP002307111 gefunden im EBI Database accession no. AX461386 & WO 01/98480 A (SYNGENTA PARTICIPATIONS AG ; BROWN DEVON (US); BUDWORTH PAUL (US); COO) 27. Dezember 2001 (2001-12-27)
- DATABASE EMBL 9. Februar 2000 (2000-02-09), "Arabidopsis thaliana oasA1 gene for O-acetylserine (thiol) lyase A1, exons 1-11." XP002307112 gefunden im EBI Database accession no. AJ272027 -& JOST R ET AL: "Genomic and functional characterization of the oas gene family encoding O-acetylserine (thiol) lyases, enzymes catalyzing the final step in cysteine biosynthesis in Arabidopsis thaliana" GENE, ELSEVIER BIOMEDICAL PRESS. AMSTERDAM, NL, Bd. 253, Nr. 2, 8. August 2000 (2000-08-08), Seiten 237-247, XP004215728 ISSN: 0378-1119
- KAUSCH ALBERT P ET AL: "Characterization and functional analysis of a bidirectional promoter from Arabidopsis." PLANT BIOLOGY (ROCKVILLE), Bd. 2001, 2001, Seite 151, XP009040251 & JOINT ANNUAL MEETINGS OF THE AMERICAN SOCIETY OF PLANT BIOLOGISTS AND THE CANADIAN SOCIETY OF PLANT; PROVIDENCE, RHODE ISLAND, USA; JULY 21-25, 2001

## Beschreibung

Die Erfindung betrifft Expressionskassetten und Vektoren, die pflanzliche bidirektionale Promotoren enthalten, sowie die Verwendung dieser Expressionskassetten oder Vektoren zur transgenen Expression von Nukleinsäuresequenzen in pflanzlichen Organismen. Die Erfindung betrifft ferner mit diesen Expressionskassetten oder Vektoren transformierte transgene pflanzliche Organismen, davon abgeleitete Kulturen, Teile oder Vermehrungsgut, sowie die Verwendung derselben zur Herstellung von Nahrungs-, Futtermitteln, Saatgut, Pharmazeutika oder Feinchemikalien.

Die Herstellung transgener Pflanzen ist eine grundlegende Technik der Pflanzenbiotechnologie und damit eine unerlässliche Voraussetzung für die pflanzliche Grundlagenforschung, sowie für die Herstellung von Pflanzen mit verbesserten, neuen Eigenschaften für die Landwirtschaft, zur Qualitätssteigerung bei Nahrungsmitteln oder zur Produktion bestimmter Chemikalien oder Pharmazeutika. Eine Grundvoraussetzung für die transgene Expression bestimmter Gene in Pflanzen ist die Bereitstellung pflanzenspezifischer Promotoren. Verschiedene pflanzliche Promotoren sind bekannt. Die derzeit in Pflanzen überwiegend verwendeten konstitutiven Promotoren sind fast ausschließlich virale oder aus *Agrobacterium* isolierte Promotoren wie beispielsweise der CaMV35S Promotor aus dem Blumenkohl-Mosaikvirus (Odell et al. (1985) Nature 313:810-812). Die zunehmende Komplexität pflanzenbiotechnologischer Arbeiten erfordert oft die Transformation mit mehreren Expressionskonstrukten. Die mehrfache Verwendung ein und desselben Promotors ist insbesondere bei Pflanzen problematisch, da das mehrfache Vorliegen gleicher regulatorischer Sequenzen eine Abschaltung der Genaktivität ("Silencing") bewirken kann (Kumpatla et al. (1998) TIBS 3:97-104; Selker (1999) Cell 97:157-160). Es besteht daher ein zunehmender Bedarf an neuen Promotoren. Ein alternativer Umgang mit diesem Problem ist die Verwendung von sogenannten "bidirektionalen" Promotoren d.h. regulatorischen Sequenzen, die in beide Richtung eine Transkription der vor- bzw. nachgeschalteten DNA Sequenzen bewirken. Hier können beispielsweise Zielgen und Markergen unter der Kontrolle einer DNA-Sequenz in eine Zelle eingebracht werden.

Die transgene Expression unter Kontrolle bidirektionaler Promotoren ist bislang kaum beschrieben. Beschrieben ist die Herstellung von bidirektionalen Promotoren aus polaren Promotoren für die Expression von Nukleinsäuren in Pflanzen mittels Fusion mit weiteren transkriptionellen Elementen (Xie M (2001) Nature Biotech 19: 677-679). Der 35S Promoter wurde ebenfalls in einen bidirektionalen Promoter umgewandelt (Dong JZ et al. (1991) BIO/TECHNOLOGY 9: 858-863). WO 02/64804 beschreibt die Konstruktion eines bidirektionalen Promotor Komplexes basierend auf der Fusion von Enhancer- und Kempromotorelementen verschiedener viraler (CaMV 35S, CsVMV) und pflanzlicher (Act2, PRb1b) Sequenzen. US20020108142 beschreibt eine regulatorische Sequenz aus einem Intron des Phosphatidylinositol Transfer-like Protein IV aus Lotus japonicus (PLP-IV; GenBank Acc.-No.: AF367434) und deren Verwendung als bidirektionaler Promoter. Dieses Intronfragment hat nur in der Infektionszone der Knöllchen eine transkriptionelle Aktivität. Andere Gewebe, Wurzeln, Blätter oder Blüten zeigen keine Färbung.

Pflanzliche Promotoren, die eine bidirektionale, ubiquitäre (d.h. im wesentlichen gewebe-unspezifische) und konstitutive Expression in Pflanzen erlauben, sind bislang nicht offenbart.

WO 03/006660 beschreibt einen Promotor eines putativen Ferredoxin Gens sowie Expressionskonstrukte, Vektoren und transgene Pflanzen, die diesen beinhalten. Die isolierten 836 bp 5'-flankierende Sequenz fusioniert mit dem Gen der Glucuronidase zeigen in transgenem Tabak überraschenderweise ein konstitutives Expressionsmuster. Die Sequenz entspricht einem Sequenzabschnitt auf Chromosom 4 von Arabidopsis thaliana wie er in der GenBank unter der Acc.-No. Z97337 hinterlegt ist (Version Z97337.2; Basenpaar 85117 bis 85952; das Gen beginnend ab bp 85953 ist mit "strong similarity to ferredoxin [2Fe-2S] I, Nostoc muscorum" annotiert). In den Antheren/Pollen der geschlossenen Blütenknospen konnte nur eine schwache Aktivität, in reifen Blüten gar keine mehr detektiert werden. Entgegen den aus den Literaturbefunden abgeleiteten Vorbehalten gegen eine Eignung des Promotors zur effektiven Expression von Selektionsmarkern (zum Beispiel aufgrund der vermuteten Blattspezifität oder der Funktion im photosynthetischen Elektronentransport), konnte eine hocheffiziente Selektion durch Kombination mit beispielsweise dem Kanamycin-Resistenzgen (nptll) demonstriert werden. In WO 03/006660 ist lediglich die Verwendung als "normaler" konstitutiver Promotor beschrieben. Eine Verwendung als bidirektionaler Promotor ist nicht offenbart.

Um über einen Transferkomplex möglichst viele Gene in ein Pflanzengenom zu integrieren, ist es notwendig, die Anzahl und Größe regulatorischer Sequenzen für die Expression transgener Nukleinsäuren zu begrenzen. Bidirektional wirkende Promotoren tragen zur Lösung dieser Aufgabe bei. Von besonderen Vorteil ist die Verwendung eines bidirektionalen Promoters, wenn dessen Aktivitäten koordiniert in gleicher Stärke vorhanden sind und auf einem kurzen DNA Fragment liegen. Da die Verwendung viraler Sequenzen für die Expression in transgenen Pflanzen wenig Akzeptanz findet, ist es von Vorteil regulatorische Sequenzen ebenfalls aus Pflanzen zu nutzen.

Die der vorliegenden Erfindung zugrunde liegende Aufgabe bestand in der Bereitstellung von transgenen Expressionskassetten umfassend pflanzliche regulatorische Sequenzen, die eine bidirektionale, ubiquitäre und entwicklungsunabhängige (konstitutive) Expression zweier transgen zu exprimierender Nukleinsäuresequenzen vermitteln.

Diese Aufgabe wird durch die vorliegende Erfindung gelöst. Ein erster Gegenstand der Erfindung betrifft daher Expressionskassetten zur transgenen Expression von zwei Nukleinsäurensequenzen in einer pflanzlichen Zelle umfassend mindestens eine regulatorische Sequenz ausgewählt aus der Gruppe bestehend aus
a) dem Promotor gemäß SEQ ID NO: 1 oder 2,
b) funktionellen Äquivalenten des Promotor gemäß SEC ID NO: 1 oder 2, die eine Identität von mindestens 80% zu der Sequenz gemäß SEQ ID NO: 1 oder 2 aufweisen und die im wesentlichen die gleiche Promotoraktivität wie der Promotor gemäß SEC ID NO: 1 oder 2 besitzen,
b) funktionellen Äquivalenten des Promotor gemäß SEQ ID NO: 1 oder 2 welche mindestens 25 aufeinanderfolgende Nukleotide der Sequenzen gemäß SEC ID NO: 1 oder 2 umfassen und die im wesentlichen die gleiche Promotoraktivität wie der Promotor gemäß SEQ ID NO: 1 oder 2 besitzen, und
c) funktionell äquivalente Fragmente der Sequenzen a) oder b) oder c), die mindestens 25 aufeinanderfolgende Nukleotide besagter Sequenzen a) oder b) oder c) aufweisen und im wesentlichen die gleiche Promotoraktivität wie der Promotor gemäß SEQ ID NO: 1 oder 2 besitzen,
wobei besagte regulatorische Sequenz zwischen zwei Nukleinsäuresequenzen angeordnet ist und in Bezug auf besagte Nukleinsäuresequenz heterolog ist und mit besagten Nukleinsäuresequenzen funktionell so verknüpft ist, dass in mindestens einer pflanzlichen Zelle die Expression von zwei unterschiedlichen Ribonukleinsäuresequenzen bewirkt wird, wobei besagte Ribonukleinsäuresequenzen ausgewählt sind aus Ribonukleinsäuresequenzen kodierend für
i) Aminosäuresequenzen oder
ii) Ribonukleinsäuresequenzen, die eine Verminderung der Expression mindestens eines endogenen Gens besagter pflanzlichen Zelle bewirken.

Ferner betrifft die Erfindung ein Verfahren zur transgenen Expression von zwei Ribonukleinsäuresequenzen in pflanzlichen Zellen, wobei eine Expressionskassetten umfassend mindestens eine regulatorische Sequenz ausgewählt aus der Gruppe bestehend aus
a) dem Promotor gemäß SEQ ID NO: 1 oder 2,
b) funktionellen Äquivalenten des Promotor gemäß SEQ ID NO: 1 oder 2, die eine Identität von mindestens 80% zu der Sequenz gemäß SEQ ID NO: 1 oder 2 aufweisen und die im wesentlichen die gleiche Promotoraktivität wie der Promotor gemäß SEQ ID NO: 1 oder 2 besitzen,
b) funktionellen Äquivalenten des Promotor gemäß SEQ ID NO: 1 oder 2 welche mindestens 25 aufeinanderfolgende Nukleotide der Sequenzen gemäß SEQ ID NO: 1 oder 2 umfassen und die im wesentlichen die gleiche Promotoraktivität wie der Promotor gemäß SEQ ID NO: 1 oder 2 besitzen, und
c) funktionell äquivalente Fragmente der Sequenzen a) oder b) oder c), die mindestens 25 aufeinanderfolgende Nukleotide besagter Sequenzen a) oder b) oder c) aufweisen und im wesentlichen die gleiche Promotoraktivität wie der Promotor gemäß SEQ ID NO: 1 oder 2 besitzen,
in mindestens eine pflanzliche Zelle eingebracht wird,
wobei besagte regulatorische Sequenz zwischen zwei Nukleinsäuresequenzen angeordnet ist und in Bezug auf besagte Nukleinsäuresequenz heterolog ist und mit besagten Nukleinsäuresequenzen funktionell so verknüpft ist, dass in mindestens besagter pflanzlichen Zelle die Expression besagter zwei unterschiedlichen Ribonukleinsäuresequenzen bewirkt wird, wobei besagte Ribonukleinsäuresequenzen ausgewählt sind aus Ribonukleinsäuresequenzen kodierend für
i) Aminosäuresequenzen oder
ii) Ribonukleinsäuresequenzen, die eine Verminderung der Expression mindestens eines endogenen Gens besagter pflanzlichen Zelle bewirken.

Die in der vorliegenden Erfindung als bidirektionaler Promotor zum Einsatz kommende DNA-Sequenz entspricht der intergenische Region zwischen einem putativen Ferredoxin (FD) Gen und einem putativen O-Acetyl-Serin-Lyase (OASTL) Gen in Arabidopsis thaliana.

Besonders gute Ergebnisse konnten in Pflanzen der Familie der Brassicaceae erzielt werden, wie beispielsweise Arabidopsis oder Raps. Aber auch in anderen Pflanzenarten (wie beispielsweise Tabak) konnten sehr gute Ergebnisse (insbesondere bei der Expression von Selektionsmarkern) erzielt werden. Die Expressionsaktivität ist im wesentlichen unabhängig von der Art der nachgeschalteten Nukleinsäure. Die Verwendung des bidirektionalen Promoters ist sowohl für die Expression von Selektionsmarkern als auch für jede andere Nukleinsäure geeignet.

In einer bevorzugten Ausführungsform sind daher die beiden in den erfindungsgemäßen Expressionskassetten umfassten transgen zu exprimierenden Nukleinsäuresequenzen bzw. die unter dem erfindungsgemäßen Verfahren exprimierten Ribonukleinsäuresequenzen unterschiedlich. "Unterschiedlich" heiß in diesem Zusammenhang, dass sich die transgen von beiden Seiten des bidirektionalen Promotors ausgehend exprimierten Ribonukleinsäuresequenzen in mindestens einer Base von einander unterscheiden. Bevorzugt kodieren beide Nukleinsäuresequenzen für unterschiedliche Proteine, bevorzugt für Proteine mit unterschiedlicher Funktion und/oder Aktivität.

Die Erfindung ermöglicht eine Erhöhung der Zahl von Transkriptionseinheiten bei einer verringerten Zahl von Promotorsequenzen. Im Fall von Translationsfusionen können auch mehr als zwei Proteine reguliert werden. Ein besonderer Vorteil dieser Erfindung ist, dass die Expression dieser multiplen Transgene unter der Kontrolle des bidirektionalen Promoters gleichzeitig und synchronisiert stattfindet. Der Promotor ist besonders gut für eine koordinierte Expression von Nukleinsäuren geeignet. So können gleichzeitig
i) Zielprotein und Selektionsmarker oder Reporterprotein
ii) Selektionsmarker und Reporterprotein
ii) Zwei Zielproteine z.B. aus dem gleichen Stoffwechselweg
iii) Sense und antisense RNA
iv) Verschiedene Proteine zur Pathogenabwehr
und vieles mehr exprimiert werden und verbesserte Effekte in den Pflanzen bewirken.

"Expression" umfasst die Transkription der transgen zu exprimierenden Nukleinsäuresequenz, kann aber - im Falle eines offenen Leserasters in "sense"-Orientierung - auch die Translation der transkribierten RNA der transgen zu exprimierenden Nukleinsäuresequenz in ein korrespondierendes Polypeptid mit einschließen.

"Expressionskassette zur transgenen Expression von Nukleinsäuren oder Verfahren zur transgenen Expression von Nukleinsäuren umfasst alle solche durch gentechnische Methoden zustande gekommene Konstruktionen oder Verfahren, in denen sich entweder
a) einer der erfindungsgemäßen Promotoren (z.B. der Promotor gemäß SEC ID NO: 1 oder 2 oder eines funktionellen Äquivalentes derselben), oder
b) die unter der Kontrolle besagten Promotors zu exprimierende Nukleinsäuresequenz, oder
c) (a) und (b)
sich nicht in ihrer natürlichen, genetischen Umgebung (d.h. an ihrem natürlichen chromosomalen Locus) befinden oder durch gentechnische Methoden modifiziert wurden, wobei die Modifikation beispielhaft eine Substitution, Addition, Deletion, Inversion oder Insertion eines oder mehrerer Nukleotidreste sein kann. Die unter Kontrolle eines der erfindungsgemäßen Promotoren zu exprimierende Nukleinsäuresequenz ist heterolog in bezug auf besagten Promotor, d.h. die steht natürlicherweise nicht unter dessen Kontrolle, sondern besagte Kontrolle wurde (beispielsweise durch gentechnische Verfahren) in nicht-natürlicher Weise hergestellt.

Die erfindungsgemäßen Expressionskassetten, von ihnen abgeleitete Vektoren oder die erfindungsgemäßen Verfahren können funktionelle Äquivalente zu den unter SEQ ID NO: 1 oder 2 beschriebenen Promotorsequenzen umfassen. Funktionell äquivalente Sequenzen umfassen auch all die Sequenzen, die von dem komplementären Gegenstrang der durch SEQ ID NO: 1 oder 2 definierten Sequenzen abgeleitet sind und im wesentlichen die gleiche Promotoraktivität aufweisen. Funktionelle Äquivalente in bezug auf die erfindungsgemäßen Promotoren meint insbesondere natürliche oder künstliche Mutationen der unter SEQ ID NO: 1 oder 2 beschriebenen Promotorsequenzen sowie deren Homologe aus anderen Pflanzengattungen und - arten, welche weiterhin im wesentlichen die gleiche Promotoraktivität aufweisen.

Eine Promotoraktivität wird im wesentlichen als gleich bezeichnet, wenn die Transkription eines bestimmten zu exprimierenden Gens unter Kontrolle eines bestimmten von SEQ ID NO: 1 oder 2 abgeleiteten Promotors unter ansonsten unveränderten Bedingungen eine Lokalisation innerhalb der Pflanze aufweist, die zu mindestens 50%, bevorzugt mindestens 70%, besonders bevorzugt mindesten 90% ganz besonders bevorzugt mindestens 95% deckungsgleich ist mit einer Vergleichsexpression erhalten unter Verwendung eines des durch SEC ID NO: 1 oder 2 beschriebenen Promotors. Dabei kann die Expressionshöhe sowohl nach unten als auch nach oben im Vergleich zu einem Vergleichswert abweichen. Bevorzugt sind dabei solche Sequenzen, deren Expressionshöhe, gemessen anhand der transkribierten mRNA oder dem infolge translatierten Protein, unter ansonsten unveränderten Bedingungen quantitativ um nicht mehr als 50%, bevorzugt 25%, besonders bevorzugt 10 % sich von einem Vergleichswert erhalten mit einem durch SEQ ID NO: 1 oder 2 beschriebenen Promotor unterscheidet. Besonders bevorzugt sind solche Sequenzen, deren Expressionshöhe, gemessen anhand der transkribierten mRNA oder dem infolge translatierten Protein, unter ansonsten unveränderten Bedingungen quantitativ um mehr als 50%, bevorzugt 100%, besonders bevorzugt 500%, ganz besonders bevorzugt 1000% eines Vergleichswert erhalten mit dem durch SEQ ID NO:1 beschriebenen Promotor übersteigt. Bevorzugt ist als Vergleichswert die Expressionshöhe der natürlichen mRNA des jeweiligen Gens oder des natürlichen Genproduktes. Bevorzugt ist ferner als Vergleichswert die Expressionshöhe erhalten mit einer beliebigen, aber bestimmten Nukleinsäuresequenz, bevorzugt solchen Nukleinsäuresequenzen, die für leicht quantifizierbare Proteine kodieren. Ganz besonders bevorzugt sind dabei Reporter-Proteine (Schenbom E & Groskreutz D (1999) Mol Biotechnol 13(1):29-44) wie das "Green Fluorescence Protein" (GFP) (Chui WL et al., Curr Biol 1996, 6:325-330; Leffel SM et al., Biotechniques. 23(5):912-8, 1997), die Chloramphenicoltransferase, eine Luziferase (Millar et al., Plant Mol Biol Rep 1992 10:324-414) oder die β-Galactosidase, ganz besonders bevorzugt ist die β-Glucuronidase (Jefferson et al. (1987) EMBO J. 6:3901-3907).

Ansonsten unveränderte Bedingungen bedeutet, dass die Expression, die durch eine der zu vergleichenden Expressionskassetten initiiert wird, nicht durch Kombination mit zusätzlichen genetischen Kontrollsequenzen, zum Beispiel Enhancer-Sequenzen, modifiziert wird. Unveränderte Bedingungen bedeutet ferner, dass alle Rahmenbeingungen wie beispielsweise Pflanzenart, Entwicklungsstadium der Pflanzen, Zuchtbedingungen, Testbedingungen (wie Puffer, Temperatur, Substrate etc.) zwischen den zu vergleichenden Expressionen identisch gehalten werden.

Mutationen umfassen Substitutionen, Additionen, Deletionen, Inversionen oder Insertionen eines oder mehrerer Nukleotidreste. Somit werden beispielsweise auch solche Nukleinsäuresequenzen durch die vorliegende Erfindung mit umfasst, welche man durch Modifikation eines Promotors gemäß SEQ ID NO: 1 oder 2 erhält. Ziel einer solchen Modifikation kann die weitere Eingrenzung der darin enthaltenen Sequenz oder z.B. auch die Einfügung weiterer Restriktionsenzymschnittstellen, die Entfernung überflüssiger DNA oder das Hinzufügen weiterer Sequenzen, zum Beispiel weiterer regulatorischer Sequenzen, sein.

Wo Insertionen, Deletionen oder Substitutionen, wie z.B. Transitionen und Transversionen, in Frage kommen, können an sich bekannte Techniken, wie in vitro-Mutagenese, "primer repair", Restriktion oder Ligation verwendet werden. Durch Manipulationen, wie z.B. Restriktion, "chewing-back" oder Auffüllen von Überhängen für "blunt ends" können komplementäre Enden der Fragmente für die Ligation zur Verfügung gestellt werden. Zu analogen Ergebnissen kann man auch unter Verwendung der Polymerasekettenreaktion (PCR) unter Verwendung spezifischer Oligonukleotid-Primer kommen.

Unter Identität zwischen zwei Nukleinsäuren wird die Identität der Nukleinsäuresequenz über die jeweils gesamte Sequenzlänge verstanden, die durch Vergleich mit Hilfe des Programmalgorithmus GAP (Wisconsin Package Version 10.0, University of Wisconsin, Genetics Computer Group (GCG), Madison, USA) unter Einstellung folgender Parameter berechnet wird:

| | |
|---|---|
| Gap Weight: 12 | Length Weight: 4 |
| Average Match: 2,912 | Average Mismatch:-2,003 |

Beispielhaft wird unter einer Sequenz, die eine Identität von mindestens 50 % auf Nukleinsäurebasis mit der Sequenz SEQ ID NO: 1 aufweist, eine Sequenz verstanden, die bei einem Vergleich mit der Sequenz SEQ ID NO: 1 nach obigem Programmalgorithmus mit obigem Parametersatz eine Identität von mindestens 50 % aufweist.

Funktionelle Äquivalente zu dem Promotor gemäß SEQ ID NO: 1 umfasst bevorzugt solche Sequenzen, die eine Identität aufweisen von mindestens 80 %, bevorzugt 90 %, besonders bevorzugt mindestens 95 %, ganz besonders bevorzugt mindestens 98 %, am meisten bevorzugt 99% zu der Sequenz gemäß SEQ ID NO: 1 und weiterhin im wesentlichen die gleiche Promotoraktivität wie die Sequenz gemäß SEC ID NO: 1 aufweisen.

Funktionelle Äquivalente zu dem Promotor gemäß SEC ID NO: 2 umfasst bevorzugt solche Sequenzen, die eine Identität aufweisen von mindestens 80 %, bevorzugt 90 %, besonders bevorzugt mindestens 95 %, ganz besonders bevorzugt mindestens 98 %, am meisten bevorzugt 99% zu der Sequenz gemäß SEQ ID NO: 2 und weiterhin im wesentlichen die gleiche Promotoraktivität wie die Sequenz gemäß SEC ID NO: 2 aufweisen.

Weitere Beispiele für die in den erfindungsgemäßen Expressionskassetten oder Vektoren zum Einsatz kommenden Promotorsequenzen lassen sich beispielsweise in verschiedenen Organismen, deren genomische Sequenz bekannt ist, wie beispielsweise aus Arabidopsis thaliana, Brassica napus, Nicotiana tabacum, Solanum tuberosum, Helianthium annuus, Linum sativum durch Identitätvergleiche in Datenbanken leicht auffinden.

Verfahren zur Herstellung erfindungsgemäßer funktioneller Äquivalente umfasst bevorzugt die Einführung von Mutationen in einen Promotor gemäß SEQ ID NO: 1. Eine Mutagenese kann ungerichtet ("random") erfolgen, wobei die mutagenisierten Sequenzen anschließend bezüglich ihrer Eigenschaften nach einer "trial-by-error" Prozedur durchmustert werden. Besonders vorteilhafte Selektionskriterien umfassen beispielsweise eine erhöht Resistenz gegenüber einem Selektionsmarker, die Höhe der resultierenden Expression der eingeführten Nukleinsäuresequenz.

In einer weiteren Ausführungsform der Erfindung können essentielle regulatorische Elemente der erfindungsgemäßen Promotoren gezielt isoliert und als solche oder in Kombination mit anderen regulatorischen Elementen eingesetzt werden. Folglich umfasst ein Gegenstand der Erfindung funktionellen Äquivalenten des Promotor gemäß SEQ ID NO: 1 oder 2 welche mindestens 25, bevorzugt mindestens 50, besonders bevorzugt mindestens 100, ganz besonders bevorzugt mindestens 200, am meisten bevorzugt mindestens 400 aufeinanderfolgende Nukleotide der Sequenzen gemäß.SEQ ID NO: 1 oder 2 umfassen und die im wesentlichen die gleiche Promotoraktivität wie der Promotor gemäß SEQ ID NO: 1 oder 2 besitzen.

Alternativ können nicht-essentielle Sequenzen eines der erfindungsgemäßen Promotors deletiert werden ohne die genannten Eigenschaften signifikant zu beeinträchtigen. Ein weiterer Gegenstand der Erfindung umfasst daher funktionell äquivalente Fragmente einer der erfindungsgemäßen Promotorsequenzen, die mindestens 25, bevorzugt mindestens 50, besonders bevorzugt mindestens 100, ganz besonders bevorzugt mindestens 200, am meisten bevorzugt mindestens 400 aufeinanderfolgende Nukleotide einer der erfindungsgemäßen Promotorsequenzen aufweisen und im wesentlichen die gleiche Promotoraktivität wie der Promotor gemäß SEC ID NO: 1 oder 2 besitzen.

Die Eingrenzung der Promotorsequenz auf bestimmte, essentielle regulatorische Regionen kann auch mit Hilfe von Suchroutine zur Suche von Promotorelementen vorgenommen werden. Oft sind in den für die Promotoraktivität relevanten Regionen bestimmte Promotorelemente gehäuft vorhanden. Diese Analyse kann beispielsweise mit Computerprogrammen wie dem Programm PLACE ("Plant Cis-acting Regulatory DNA Elements") vorgenommen werden (Higo K et al. (1999) Nucleic Acids Res 27:1, 297-300) oder der BIOBASE Datenbank "Transfac" (Biologische Datenbanken GmbH, Braunschweig).

Verfahren zur Mutagenisierung von Nukleinsäuresequenzen sind dem Fachmann bekannt und schließen beispielhaft die Verwendung von Oligonukleotiden mit einer oder mehr Mutationen im Vergleich zu der zu mutierenden Region ein (z.B. im Rahmen einer "Site-specific mutagenesis"). Typischerweise kommen Primer mit ungefähr 15 bis ungefähr 75 Nukleotiden oder mehr zum Einsatz, wobei bevorzugt ca. 10 bis ca. 25 oder mehr Nukleotidreste an beiden Seiten der zu verändernden Sequenz lokalisiert sind. Details und Durchführung besagter Mutageneseverfahren sind dem Fachmann geläufig (Kunkel et al. (1987) Methods Enzymol 154:367-382; Tomic et al. (1990) Nucl Acids Res 12:1656; Upender et al. (1995) Biotechniques 18(1):29-30; US 4,237,224). Eine Mutagenese kann auch durch Behandlung von beispielsweise Vektoren, die eine der erfindungsgemäßen Nukleinsäuresequenzen enthalten, mit mutagenisierenden Agentien wie Hydroxylamin realisiert werden.

Die in den erfindungsgemäßen Expressionskassetten enthaltenen transgen zu exprimierenden Nukleinsäuresequenzen können mit weiteren genetischen Kontrollsequenzen neben einem der erfindungsgemäßen Promotoren funktionell verknüpft sein.

Unter einer funktionellen Verknüpfung versteht man zum Beispiel die sequentielle Anordnung eines Promotors, der transgen zu exprimierenden Nukleinsäuresequenz und ggf. weiterer regulativer Elemente wie zum Beispiel einem Terminator derart, dass jedes der regulativen Elemente seine Funktion bei der transgenen Expression der Nukleinsäuresequenz, je nach Anordnung der Nukleinsäuresequenzen zu sense oder anti-sense RNA, erfüllen kann. Dazu ist nicht unbedingt eine direkte Verknüpfung im chemischen Sinne erforderlich. Genetische Kontrollsequenzen, wie zum Beispiel Enhancer-Sequenzen, können ihre Funktion auch von weiter entfernten Positionen oder gar von anderen DNA-Molekülen aus auf die Zelsequenz ausüben. Bevorzugt sind Anordnungen, in denen die transgen zu exprimierenden Nukleinsäuresequenz hinter der als Promotor fungierenden Sequenz positioniert wird, so dass beide Sequenzen kovalent miteinander verbunden sind. Bevorzugt ist dabei der Abstand zwischen der Promotorsequenz und der transgen zu exprimierende Nukleinsäuresequenz geringer als 200 Basenpaare, besonders bevorzugt kleiner als 100 Basenpaare, ganz besonders bevorzugt kleiner als 50 Basenpaare.

Die Herstellung einer funktionellen Verknüpfung kann mittels gängiger Rekombinations- und Klonierungstechniken realisiert werden, wie sie beispielsweise in Maniatis T et al. (1989) Molecular Cloning: A Laboratory Manual, Cold Spring Harbor Laboratory, Cold Spring Harbor, NY sowie in Silhavy TJ et al. (1984) Experiments with Gene Fusions, Cold Spring Harbor Laboratory, Cold Spring Harbor, NY und in Ausubel FM et al.(1987) Current Protocols in Molecular Biology, Greene Publishing Assoc. and Wiley Interscience beschrieben sind. Zwischen beide Sequenzen können aber auch weitere Sequenzen positioniert werden, die zum Beispiel die Funktion eines Linkers mit bestimmten Restriktionsenzymschnittstellen oder eines Signalpeptides haben. Auch kann die Insertion von Sequenzen zur Expression von Fusionsproteinen führen.

Der Begriff der genetischen Kontrollsequenzen ist breit zu verstehen und meint all solche Sequenzen, die einen Einfluss auf das Zustandekommen oder die Funktion der erfindungsgemäßen Expressionskassette haben. Genetische Kontrollsequenzen modifizieren zum Beispiel die Transkription und Translation in prokaryotischen oder eukaryotischen Organismen. Vorzugsweise umfassen die erfindungsgemäßen Expressionskassetten 5'-stromaufwärts von der jeweiligen transgen zu exprimierenden Nukleinsäuresequenz einen der erfindungsgemäßen Promotoren und 3'-stromabwärts eine Terminatorsequenz als zusätzliche genetische Kontrollsequenz, sowie gegebenenfalls weitere übliche regulative Elemente, und zwar jeweils funktionell verknüpft mit der transgen zu exprimierenden Nukleinsäuresequenz.

Genetische Kontrollsequenzen umfassen auch weitere Promotoren, Promotorelemente oder Minimalpromotoren, die die expressionsteuemden Eigenschaften modifizieren können. So kann durch genetische Kontrollsequenzen zum Beispiel die gewebespezifische Expression zusätzlich abhängig von bestimmten Stressfaktoren erfolgen. Entsprechende Elemente sind zum Beispiel für Wasserstress, Abscisinsäure (Lam E und Chua NH, (1991) J Biol Chem 266(26):17131-17135) und Hitzestress (Schöffl F et al. (1989) Mol Gen Genetics 217(2-3):246-53) beschrieben.

Es können ferner weitere Promotoren funktionell mit der zu exprimierenden Nukleinsäuresequenz verknüpft sein, die eine Expression in weiteren Pflanzengeweben oder in anderen Organismen, wie zum Beispiel *E.coli* Bakterien ermöglichen. Als Pflanzenpromotoren kommen im Prinzip alle oben beschriebenen Promotoren in Frage. Vorstellbar ist zum Beispiel, dass eine bestimmte Nukleinsäuresequenz durch einen Promotor (zum Beispiel einen der erfindungsgemäßen Promotoren) in einem Pflanzengewebe als sense-RNA transkribiert und in das entsprechende Protein translatiert wird, während die gleiche Nukleinsäuresequenz durch einen anderen Promotor mit einer anderen Spezifität in einem anderen Gewebe zu anti-sense-RNA transkribiert und das entsprechende Protein herunterreguliert wird. Dieses kann durch eine erfindungsgemäße Expressionskassette realisiert werden, indem der eine Promotor vor die transgen zu exprimierende Nukleinsäuresequenz positioniert wird und der andere Promotor dahinter.

Genetische Kontrollsequenzen umfassen ferner auch die 5'-untranslatierte Region, Introns oder die nichtkodierende 3'-Region von Genen, bevorzugt des pFD Genes und/oder des OASTL Genes. Es ist gezeigt worden, dass untranslatierte Regionen eine signifikante Funktionen bei der Regulation der Genexpression spielen können. So wurde gezeigt, dass 5'-untranslatierte Sequenzen die transiente Expression heterologer Gene verstärken können. Sie können ferner die Gewebespezifität fördern (Rouster J et al.(1998) Plant J. 15:435-440.). Umgekehrt unterdrückt die 5'-untranslatierte Region des opaque-2 Gens die Expression. Eine Deletion der entsprechenden Region führt zu einer Erhöhung der Genaktivität (Lohmer S et al. (1993) Plant Cell 5:65-73). Die unter SEC ID NO: 2 angegebene Nukleinsäuresequenz enthält den Abschnitt des FD-Gens und des OASTL Gens, der den Promotor und die 5'-untranslatierte Region bis vor das ATG-Startcodon des jeweiligen Proteins repräsentiert. In der 5' untranslatierten Region des OASTL Gens befindet sich ein Intron, was durch die Struktur der cDNA Klone belegt werden kann. Die Introngrenzen liegen bei 14 bp (3' Seite des Introns) und 281 bp (5'Seite des Introns). Basenpaar Nummerierung entsprechend des Nummerierung des Promotors gemäß SEC ID NO: 2. Das Intron hat eine starke expressionsfördemde Funktion in beide Transkriptionsrichtungen. Dies könnte durch die Existenz eines Enhancers in dieser Region begründet sein.

In einer bevorzugten Ausführungsform ist daher der erfindungsgemäß0e bidirektionale Promotor beschrieben durch die Sequenz gemäß SEC ID NO: 2 oder durch Sequenzen die eine Identität von mindestens 80%, bevorzugt mindestens 90%, besonders bevorzugt mindestens 95%, ganz besonders bevorzugt mindesten 98%, am meisten bevorzugt mindestens 99% zu der Sequenz gemäß SEC ID NO: 2 haben.

Weitere 5'-untranslatierte Sequenzen sowie Introns mit expressionsförderender Funktion sind dem Fachmann bekannt McElroy und Mitarbeiter (McElroy et al. (1991) Mol Gen Genet 231(1):150-160) berichteten von einem auf dem Reis Actin 1 (Act1) Promotor basierenden Konstrukt zur Transformation monokotyler Pflanzen. In transgenen Reiszellen führte die Verwendung des Act1-Introns in Kombination mit dem 35S-Promotor zu einer gegenüber dem isolierten 35S-Promotor um das Zehnfache gesteigerten Expressionsrate. Eine Optimierung der Sequenzumgebung der Translations-Initiationsstelle des Reportergen-Gens (GUS) resultierte in einer vierfachen Steigerung der GUS-Expression in transformierten Reiszellen. Eine Kombination der optimierten Translations-Initiationsstelle und des Actl-Introns resultierte in einer 40-fachen Steigerung der GUS-Expression durch den CaMV35S-Promotor in transformierten Reiszellen; ähnliche Ergebnisse wurden anhand von transformierten Maiszellen erzielt. Insgesamt wurde aus den zuvor beschriebenen Untersuchungen geschlussfolgert, dass die auf dem Act1-Promotor basierenden Expressionsvektoren dazu geeignet sind, eine hinreichend starke und konstitutive Expression von Fremd-DNA in transformierten Zellen monokotyler Pflanzen zu steuern.

Die Expressionskassette kann eine oder mehrere sogenannte "Enhancer"-Sequenzen funktionell verknüpft mit dem Promotor enthalten, die eine erhöhte transgene Expression der Nukleinsäuresequenz ermöglichen. Auch am 3'-Ende der transgen zu exprimierenden Nukleinsäuresequenzen können zusätzliche vorteilhafte Sequenzen inseriert werden, wie weitere regulatorische Elemente oder Terminatoren. Die transgen zu exprimierenden Nukleinsäuresequenzen können in einer oder mehreren Kopien in einer der erfindungsgemäßen Expressionskassetten enthalten sein.

Als Kontrollsequenzen sind weiterhin solche zu verstehen, die eine homologe Rekombination bzw. Insertion in das Genom eines Wirtsorganismus ermöglichen oder die Entfernung aus dem Genom erlauben. Bei der homologen Rekombination kann zum Beispiel der natürliche Promotor eines bestimmten Gens gegen einen der erfindungsgemäßen Promotoren ausgetauscht werden. Methoden wie die cre/lox-Technologie erlauben eine gewebespezifische, unter Umständen induzierbare Entfernung der Expressionskassette aus dem Genom des Wirtsorganismus (Sauer B. (1998) Methods. 14(4):381-92). Hier werden bestimmte flankierende Sequenzen dem Zielgen angefügt (lox-Sequenzen), die später eine Entfernung mittels der cre-Rekombinase ermöglichen.

Der einzuführende Promotor kann mittels homologer Rekombination vor das transgen zu exprimierende Zielgen platziert werden, indem der Promotor mit DNA-Sequenzen verknüpft wird, die zum Beispiel zu endogenen Sequenzen homolog sind, die dem Leseraster des Zielgens vorgelagert sind. Derartige Sequenzen sind als genetische Kontrollsequenzen zu verstehen. Nachdem eine Zelle mit dem entsprechenden DNA-Konstrukt transformiert wurde, können die beiden homologen Sequenzen interagieren und so die Promotorsequenz an dem gewünschten Ort vor dem Zielgen platzieren, so dass die Promotorsequenz nun in funktioneller Verknüpfung mit dem Zielgen steht und eine erfindungsgemäße Expressionskassette bildet. Die Auswahl der homologen Sequenzen bestimmt den Insertionsort des Promotors. Hierbei kann die Expressionskassette durch homologe Rekombination mittels einer einfachen oder einer doppelten-reziproken Rekombination generiert werden. Bei der einfach reziproken Rekombination wird nur eine einzelne Rekombinationssequenz verwendet und es erfolgt die Insertion der gesamten eingeführten DNA. Bei der doppelt-reziproken Rekombination ist die einzuführende DNA durch zwei homologe Sequenzen flankiert und es erfolgt die Insertion des flankierten Bereiches. Letzteres Verfahren ist geeignet, um wie oben beschrieben den natürlichen Promotor eines bestimmten Gens gegen einen der erfindungsgemäßen Promotoren auszutauschen und so den Expressionsort und -zeitpunkt dieses Gens zu modifizieren. Diese funktionelle Verknüpfung stellt eine erfindungsgemäße Expressionskassette dar.

Zur Selektion erfolgreich homolog rekombinierter oder auch transformierter Zellen ist es in der Regel erforderlich, einen selektionierbaren Marker zusätzlich einzuführen. Verschiedene geeignete Marker sind unten genannt. Der Selektionsmarker erlaubt die Selektion der transformierten Zellen von untransformierten. Homologe Rekombination ist ein relativ seltenes Ereignis in höheren Eukaryoten, vor allem in Pflanzen. Zufällige Integrationen in das Wirtsgenom überwiegen. Eine Möglichkeit die zufällig integrierten Sequenzen zu entfernen und so Zellklone mit einer korrekten homologen Rekombination anzureichern, besteht in der Verwendung eines sequenzspezifischen Rekombinationssystems wie in US 6,110,736 beschrieben.

Als Kontrollsequenzen geeignete Polyadenylierungssignale sind pflanzliche Polyadenylierungssignale, sowie - vorzugsweise - solche aus *Agrobacterium tumefaciens.* In einer besonders bevorzugten Ausführungsform enthält die Expressionskassette eine in Pflanzen funktionelle Terminatorsequenz. In Pflanzen funktionelle Terminatorsequenzen meint allgemein solche Sequenzen, die in der Lage sind, in Pflanzen den Abbruch der Transkription einer DNA-Sequenz zu bewirken. Beispiele für geeignete Terminatorsequenzen sind der OCS (Octopin-Synthase)-Terminator und der NOS (Nopalin-Synthase)-Terminator. Besonders bevorzugt sind jedoch pflanzliche Terminatorsequenzen. Pflanzliche Terminatorsequenzen meint allgemein solche Sequenzen, die Bestandteil eines natürlichen pflanzlichen Gens sind. Besonders bevorzugt sind dabei der Terminators des Cathepsin D Inhibitor Gens aus Kartoffel (GenBank Acc. No.: X74985) oder des Terminators der Speicherproteingens VfLEIB3 (GenBank Acc. No.: Z26489) aus der Ackerbohne. Diese Terminatoren sind den im Stand der Technik beschriebenen viralen oder T-DNA Terminatoren mindestens gleichwertig.

Dem Fachmann ist eine Vielzahl von Nukleinsäuren bzw. Proteinen bekannt, deren rekombinante Expression gesteuert durch die erfindungsgemäßen Expressionskassetten oder Verfahren vorteilhaft ist. Ferner sind dem Fachmann eine Vielzahl von Genen bekannt, durch deren Reprimierung oder Ausschaltung mittels Expression einer entsprechenden antisense-RNA ebenfalls vorteilhafte Effekte erreicht werden können. Beispielhaft jedoch nicht einschränkend für vorteilhafte Effekte seien zu nennen:
- Erleichterte Herstellung eines transgenen Organismus beispielsweise durch die Expression von Selektionsmarkern
- Erzielen einer Resistenz gegen abiotische Stressfaktoren (Hitze, Kälte, Trockenheit, erhöhte Feuchtigkeit, Umweltgifte, UV-Strahlung)
- Erzielen einer Resistenz gegen biotische Stressfaktoren (Pathogene, Viren, Insekten und Krankheiten)
- Verbesserung von Nahrungs- oder Futtereigenschaften
- Verbesserung der Wachstumsrate oder des Ertrages.

Nachfolgend seien einige konkrete Beispiele für Nukleinsäuren genannt, deren Expression die gewünschten vorteilhaften Effekte bietet:
1. Selektionsmarker
   Selektionsmarker umfasst sowohl positive Selektionsmarker, die eine Resistenz gegen ein Antibiotikum, Herbizid oder Biozid verleihen, als auch negative Selektionsmarker, die eine Sensitivität gegen eben diese verleihen, als auch Marker die dem transformierten Organismus einen Wachstumsvorteil gewähren (beispielsweise durch Expression von Schlüsselgenen der Cytokinbiosynthese; Ebinuma H et al. (2000) Proc Natl Acad Sci USA 94:2117-2121). Bei der positiven Selektion gedeihen nur die Organismen, die den entsprechenden Selektionsmarker exprimieren, während bei der negativen Selektion eben diese eingehen. Bei der Herstellung transgener Pflanzen ist die Verwendung eines positiven Selektionsmarkers bevorzugt. Bevorzugt ist ferner die Verwendung von Selektionsmarkern, die Wachstumsvorteile verleihen. Negative Selektionsmarker können vorteilhaft verwendet werden, wenn es darum geht, bestimmte Gene oder Genomabschnitte aus einem Organismus zu entfernen (beispielsweise im Rahmen eines Kreuzungsprozesses).
   Der mit der Expressionskassette eingebrachte selektionierbare Marker verleiht den erfolgreich rekombinierten oder transformierten Zellen eine Resistenz gegen ein Biozid (zum Beispiel ein Herbizid wie Phosphinothricin, Glyphosat oder Bromoxynil), einen Metabolismusinhibitor wie 2-Desoxyglucose-6-phosphat (WO 98/45456) oder ein Antibiotikum, wie zum Beispiel Kanamycin, G 418, Bleomycin, Hygromycin. Der Selektionsmarker erlaubt die Selektion der transformierten Zellen von untransformierten (McCormick et al. (1986) Plant Cell Rep 5:81-84). Besonders bevorzugte Selektionsmarker sind solche die eine Resistenz gegen Herbizide verleihen. Dem Fachmann sind zahlreiche derartiger Selektionsmarker und die für diese kodierenden Sequenzen bekannt. Nachfolgend seien beispielhaft jedoch nicht einschränkend zu nennen:
   i) Positive Selektionsmarker:
      Der mit der Expressionskassette eingebrachte selektionierbare Marker verleiht der erfolgreich transformierten Zellen eine Resistenz gegen ein Biozid (zum Beispiel ein Herbizid wie Phosphinothricin, Glyphosat oder Bromoxynil), einen Metabolismusinhibitor wie 2-Desoxyglucose-6-phosphat (WO 98/45456) oder ein Antibiotikum, wie zum Beispiel Tetracyclin, Ampicillin, Kanamycin, G 418, Neomycin, Bleomycin oder Hygromycin. Der Selektionsmarker erlaubt die Selektion der transformierten Zellen von untransformierten (McCormick et al. (1986) Plant Cell Reports 5:81-84). Besonders bevorzugte Selektionsmarker sind solche die eine Resistenz gegen Herbizide verleihen. Beispielhaft als Selektionsmarker seien genannt:
      - DNA Sequenzen, die für Phosphinothricinacetyltransferasen (PAT; auch Bialophos-Resistenzgen (bar) genannt) kodieren und eine Detoxifizierung des Herbizids Phosphinothricin (PPT) bewirken (de Block et al. (1987) EMBO J 6:2513-2518). Geeignete bar Gene können aus beispielsweise Streptomyces hygroscopicus oder S. viridochromogenes isoliert werden. Entsprechende Sequenzen sind dem Fachmann bekannt (GenBank Acc.-No.: X17220, X05822, M22827, X65195; US 5,489,520). Ferner sind synthetische Gene beispielsweise für die Expression in Plastiden beschrieben AJ028212. Ein synthetisches Pat Gen ist beschrieben bei Becker et al. (1994) Plant J 5:299-307. Die Gene verleihen Resistenz gegen das Herbizid Bialaphos und sind ein vielbenutzter Marker in transgenen Pflanzen (Vickers JE et al. (1996) Plant Mol Biol Rep 14:363-368; Thompson CJ et al. (1987) EMBO J 6:2519-2523).
      - 5-Enolpyruvylshikimat-3-phosphatsynthasegene (EPSP Synthasegene), die eine Resistenz gegen Glyphosat (N-(phosphonomethyl)glycin) verleihen (Steinrucken HC et al. (1980) Biochem Biophys Res Commun 94:1207-1212; Levin JG und. Sprinson DB (1964) J Biol Chem 239:1142-1150; Cole DJ (1985) Mode of action of glyphosate; A literature analysis, p. 48-74. In: Grossbard E und Atkinson D (eds.). The herbicide glyphosate. Buttersworths, Boston.). Glyphosat-tolerante EPSPS Varianten werden bevorzugt als Selektionsmarker verwendet (Padgette SR et al. (1996). New weed control opportunities: development of soybeans with a Roundup Ready™ gene. In: Herbicide Resistant Crops (Duke SO ed.), pp. 53-84. CRC Press, Boca Raton, FL; Saroha MK und Malik VS (1998) J Plant Biochem Biotechnol 7:65-72). Das EPSPS Gen des Agrobacterium sp. strain CP4 hat eine natürliche Toleranz gegen Glyphosat, die auf entsprechende transgene Pflanzen transferiert werden kann (Padgette SR et al.(1995) Crop Science 35(5):1451-1461). 5-Enolpyrvylshikimate-3-phosphate-synthasen, die Glyphosat-tolerant sind, sind beispielsweise beschrieben in US 5,510,471; US 5,776,760; US 5,864,425; US 5,633;435; US 5,627;061; US 5,463,175; EP 0 218 571. Weitere Sequenzen sind beschrieben unter GenBank Accession X63374. Ferner ist das aroA Gen bevorzugt (M10947).
      - das für das Glyphosat degradierende Enzyme kodierende gox Gen (Glyphosatoxidoreduktase aus Achromobacter sp.). GOX kann eine Resistenz gegen Glyphosat vermitteln (Padgette SR et al. (1996) J Nutr.126(3):702-16; Shah D et al. (1986) Science 233: 478-481).
      - das deh Gen (kodierend für eine Dehalogenase, die Dalapon inaktiviert), (GenBank Acc.-No.: AX022822, AX022820 sowie WO99/27116)
      - bxn Gene, die für Bromoxynil degradierende Nitrilaseenzyme kodieren. Beispielsweise die Nitrilase aus Klebsiella ozanenae. Sequenzen sind in der GenBank beispielsweise unter den Acc.-No: E01313 und J03196 zu finden.
      - Neomycinphosphotransferasen verleihen eine Resistenz gegen Antibiotika (Aminoglykoside) wie Neomycin, G418, Hygromycin, Paromomycin oder Kanamycin, indem sie durch eine Phosphorylierungsreaktion deren inhibierende Wirkung reduzieren. Besonders bevorzugt ist das nptll Gen. Sequenzen können aus der GenBank erhalten werden (AF080390 Minitransposon mTn5-GNm; AF080389 Minitransposon mTn5-Nm, complete sequence). Zudem ist das Gen bereits Bestandteil zahlreicher Expressionsvektoren und kann unter Verwendung von dem Fachmann geläufigen Verfahren (wie beispielsweise Polymerasekettenreaktion) aus diesen isoliert werden (AF234316 pCAMBIA-2301; AF234315 pCAMBIA-2300, AF234314 pCAMBIA-2201). Das NPTII Gen kodiert für eine Aminoglycosid-3'O-phosphotransferase aus E.coli, Tn5 (GenBank Acc.-No: U00004 Position 1401-2300; Beck et al. (1982) Gene 19 327-336).
      - das DOG^{R}1-Gen. Das Gen DOG^{R}1 wurde aus der Hefe Saccharomyces cerevisiae isoliert (EP 0 807 836). Es codiert für eine 2-Desoxyglukose-6-phosphat Phosphatase, die Resistenz gegenüber 2-DOG verleiht (Randez-Gil et al. 1995, Yeast 11, 1233-1240; Sanz et al. (1994) Yeast 10:1195- 1202, Sequenz: GenBank Acc.-No.: NC001140 chromosom VIII, Saccharomyces cervisiae Position 194799-194056).
      - Sulfonylhamstoff- und Imidazolinon inaktivierende Acetolactatsynthasen, die eine Resistenz gegen ImidazolinoNSulfonylharnstoff-Herbizide verleihen. Geeignet sind beispielsweise die unter der GenBank Acc-No.: X51514 hinterabgelegte Sequenz für das Arabidopsis thaliana Csr 1.2 Gen (EC 4.1.3.18) (Sathasivan K et al. (1990) Nucleic Acids Res. 18(8):2188). Acetolactatesynthasen die eine Resistenz gegen Imidazolinon-Herbizide verleihen sind ferner beschrieben unter den GenBank Acc.-No.: AB049823, AF094326, X07645, X07644, A19547, A19546, A19545, 105376, 105373, AL133315.
      - Hygromycinphosphotransferasen (X74325 P. pseudomallei gene for hygromycin phosphotransferase) die eine Resistenz gegen das Antibiotikum Hygromycin verleihen. Das Gen ist Bestandteil zahlreicher Expressionsvektoren und kann unter Verwendung von dem Fachmann geläufigen Verfahren (wie beispielsweise Polymerasekettenreaktion) aus diesen isoliert werden (AF294981 pINDEX4; AF234301 pCAMBIA-1380; AF234300 pCAMBIA-1304; AF234299 pCAMBIA-1303; AF234298 pCAMBIA-1302; AF354046 pCAMBIA-1305.; AF354045 pCAMBIA-1305.1)
      - Resistenzgene gegen
         a) Chloramphenicol (Chloramphenicolacetyltransferase),
         b) Tetracyclin, verschiedene Resistenzgene sind beschrieben z.B. X65876 S. ordonez genes class D tetA and tetR for tetracycline resistance and repressor proteins X51366 Bacillus cereus plasmid pBC16 tetracycline resistance gene. Zudem ist das Gen bereits Bestandteil zahlreicher Expressionsvektoren und kann unter Verwendung von dem Fachmann geläufigen Verfahren (wie beispielsweise Polymerasekettenreaktion) aus diesen isoliert werden
         c) Streptomycin, verschiedene Resistenzgene sind beschrieben z.B. mit der GenBank Acc.-No.:AJ278607 Corynebacterium acetoacidophilum ant gene for streptomycin adenylyltransferase.
         d) Zeocin, das entsprechende Resistenzgen ist Bestandteil zahlreicher Klonierungsvektoren (z.B. L36849 Cloning vector pZEO) und kann unter Verwendung von dem Fachmann geläufigen Verfahren (wie beispielsweise Polymerasekettenreaktion) aus diesen isoliert werden.
         e) Ampicillin (β-Lactamase Gen; Datta N, Richmond MH.(1966) Biochem J. 98(1):204-9; Heffron F et al (1975) J. Bacteriol 122: 250-256; das Amp Gen wurde zuerst zur Herstellung des E. coli Vektors pBR322 kloniert, Bolivar F et al. (1977) Gene 2:95-114). Die Sequenz ist Bestandteil zahlreicher Klonierungsvektoren und kann unter Verwendung von dem Fachmann geläufigen Verfahren (wie beispielsweise Polymerasekettenreaktion) aus diesen isoliert werden.
      - Gene wie die lsopentenyltransferase aus Agrobacterium tumefaciens (strain:PO22) (Genbank Acc.-No.: AB025109). Das ipt Gen ist ein Schlüsselenzym der CytokinBiosynthese. Seine Überexpression erleichtert die Regeneration von Pflanzen (z.B. Selektion auf Cytokin-freiem Medium). Das Verfahren zur Nutzung des ipt Gens ist beschrieben (Ebinuma H et al. (2000) Proc Natl Acad Sci USA 94:2117-2121; Ebinuma H *et* al. (2000) Selection of Marker-free transgenic plants using the oncogenes (*ipt, rol* A, B, C) of Agrobacterium as selectable markers, In Molecular Biology of Woody Plants. Kluwer Academic Publishers).

      Verschiedene weitere positive Selektionsmarker, die den transformierten Pflanzen einen Wachstumsvorteil gegenüber nicht-transformierten verleihen, sowie Verfahren zu ihrer Verwendung sind u.a. beschrieben in EP-A 0 601 092. Beispielhaft sind zu nennen β-Glucuronidase (in Verbindung mit z.B. Cytokininglucuronid), Mannose-6-phosphat-Isomerase (in Verbindung mit Mannose), UDP-Galaktose-4-Epimerase (in Verbindung mit z.B. Galactose), wobei Mannose-6-phosphat-Isomerase in Verbindung mit Mannose besonders bevorzugt ist.
   ii) Negative Selektionsmarker
      Negative Selektionsmarker ermöglichen beispielsweise die Selektion von Organismen mit erfolgreich deletierten Sequenzen, die das Markergen umfassen (Koprek T et al. (1999) Plant J 19(6):719-726).Bei der negativen Selektion wird beispielsweise durch den in die Pflanze eingebrachten negativen Selektionsmarker eine Verbindung, die ansonsten für die Pflanze keine nachteilige Wirkung hat, in eine Verbindung mit nachteiliger Wirkung umgesetzt. Ferner sind Gene geeignet, die per se eine nachteilige Wirkung haben, wie zum Beispiel Thymidinkinase (TK), Diphtheria Toxin A Fragment (DT-A), das codA Genprodukt kodierend für eine Cytosindeaminase (Gleave AP et al. (1999) Plant Mol Biol. 40(2):223-35; Perera RJ et al. (1993) Plant Mol. Biol 23(4): 793-799; Stougaard J (1993) Plant J 3:755-761), das Cytochrom P450 Gen (Koprek et al. (1999) Plant J 16:719-726), Gene kodierend für eine Haloalkan Dehalogenase (Naested H (1999) Plant J 18:571-576), das iaaH Gen (Sundaresan V et al. (1995) Genes & Development 9:1797-1810) oder das tms2 Gen (Fedoroff NV & Smith DL (1993) Plant J 3:273-289).
      Die jeweils für die Selektion verwendeten Konzentrationen der Antibiotika, Herbizide, Biozide oder Toxine müssen an die jeweiligen Testbedingungen bzw. Organismen angepasst werden. Beispielhaft seien für Pflanzen zu nennen Kanamycin (Km) 50 mg/l, Hygromycin B 40 mg/l, Phosphinothricin (Ppt) 6 mg/l.
      Ferner können funktionelle Analoga der genannten Nukleinsäuren kodierend für Selektionsmarker exprimiert werden. Funktionelle Analoga meint hier all die Sequenzen, die im wesentlichen die gleiche Funktion haben d.h. zu einer Selektion transformierter Organismen befähigt sind. Dabei kann das funktionelle Analogon sich in anderen Merkmalen durchaus unterscheiden. Es kann zum Beispiel eine höhere oder niedrigere Aktivität haben oder auch über weitere Funktionalitäten verfügen.
2. Verbesserter Schutz der Pflanze gegen abiotische Stressfaktoren wie Trockenheit, Hitze, oder Kälte zum Beispiel durch Überexpression von "antifreeze"- Polypeptiden aus Myoxocephalus Scorpius (WO 00/00512), Myoxocephalus octodecemspinosus, dem Arabidopsis thaliana Transkriptionsaktivator CBF1, Glutamatdehydrogenasen (WO 97/12983, WO 98/11240), Calcium-abhängigen Proteinkinasegenen (WO 98/26045), Calcineurinen (WO 99/05902), Famesyltransferasen (WO 99/06580), Pei ZM et al., Science 1998, 282: 287-290), Ferritin (Deak M et al., Nature Biotechnology 1999, 17:192-196), Oxalatoxidase (WO 99/04013; Dunwell JM Biotechnology and Genetic Engeneering Reviews 1998, 15:1-32), DREB1A-Faktor (dehydration response element B 1A; Kasuga M et al., Nature Biotechnology 1999, 17:276-286), Genen der Mannitol- oder Trehalosesynthese wie der Trehalosephosphatsynthase oder der Trehalosephosphatphosphatase (WO 97/42326), oder durch Inhibition von Genen wie der Trehalase (WO 97/50561). Besonders bevorzugt sind Nukleinsäuren, die für den transkriptionellen Aktivator CBF1 aus Arabidopsis thaliana (GenBank Acc.-No.: U77378) oder das "antifreeze protein" aus Myoxocephalus octodecemspinosus (GenBank Acc.-No.: AF306348) oder funktionelle Äquivalente derselben kodieren.
3. Expression von Stoffwechselenzymen zur Verwendung im Futter- und Nahrungsmittelbereich, zum Beispiel die Expression von Phytase und Cellulasen. Besonders bevorzugt sind Nukleinsäuren wie die künstliche für eine mikrobielle Phytase kodierende cDNA (GenBank Acc.-No.: A19451) oder funktionelle Äquivalente derselben.
4. Erreichen einer Resistenz zum Beispiel gegen Pilze, Insekten, Nematoden und Krankheiten durch gezielte Absonderung oder Anreicherung bestimmter Metaboliten oder Proteine in der Epidermis des Embryos. Beispielhaft seien genannt Glucosinolate (Abwehr von Herbivoren), Chitinasen oder Glucanasen und andere Enzyme die die Zellwand von Parasiten zerstören, Ribosom-inaktivierende Proteine (RIPs) und andere Proteine der pflanzlichen Resistenz- und Stressreaktion, wie sie bei Verletzung oder mikrobiellem Befall von Pflanzen oder chemisch durch zum Beispiel Salicylsäure, Jasmonsäure oder Ethylen induziert werden, Lysozyme aus nicht-pflanzlichen Quellen wie zum Beispiel T4 Lysozym oder Lysozym aus verschiedenen Säugern, insektizide Proteine wie *Bacillus thuringiensis* Endotoxin, α-Amylaseinhibitor oder Proteaseinhibitoren (cowpea Trypsininhibitor), Glucanasen, Lectinen wie Phytohemagglutinin, Weizenkeimagglutinin, RNAsen oder Ribozyme. Besonders bevorzugt sind Nukleinsäuren, die für die chit42 Endochitinase aus Trichoderma harzianum (GenBank Acc.-No.: S78423) oder für das N-hydroxylierende, multifunktionelle Cytochrom P-450 (CYP79) Proteine aus Sorghum bicolor (GenBank Acc.-No.: U32624) oder funktionelle Äquivalente derselben kodieren.
5. Bekannt ist die Akkumulation von Glucosinolaten in Pflanzen der Gattung der Cardales insbesondere der Ölsaaten zum Schutz vor Schädlingen (Rask L et al.(2000) Plant Mol Biol 42:93-113; Menard R et al. (1999) Phytochemistry 52:29-35), die Expression des *Bacillus thuringiensis* Endotoxin unter Kontrolle des 35 S CaMV Promotors (Vaeck et al. (1987) Nature 328:33-37) oder der Schutz des Tabaks gegen Pilzbefall durch Expression einer Chitinase aus der Bohne unter Kontrolle des CaMV Promotors (Broglie et al. (1991) Science 254:1194-119.
   Die Expression synthetischer crylA(b) and crylA(c) Gene, die für Lepidopterenspezifische delta-Endotoxine aus *Bacillus thuringiensis* kodieren, kann in verschiedenen Pflanzen eine Resistenz gegen Schadinsekten bewirken. So kann in Reis eine Resistenz gegen zwei der wichtigsten Reisschädlinge, den gestreiften Stengelbohrer (*Chilo suppressalis*) und den gelben Stengelbohrer (*Scirpophaga incertulas*), erzielt werden (Cheng X et al. (1998) Proc Natl Acad Sci USA 95(6):2767-2772; Nayak P et al. (1997) Proc Natl Acad Sci USA 94(6):2111-2116).
5. Expression von Genen, die eine Akkumulation von Feinchemikalien, wie von Tocopherolen, Tocotrienolen oder Carotinoiden bewirken. Beispielhaft sei die Phytoendesaturase genannt. Bevorzugt sind Nukleinsäuren, die für die Phytoendesaturase aus Narcissus pseudonarcissus (GenBank Acc.-No.: X78815) oder funktionelle Äquivalente derselben kodieren.
6. Produktion von Neutraceuticals wie zum Beispiel polyungesättigten Fettsäuren wie beispielsweise Arachidonsäure oder EP (Eicosapentaensäure) oder DHÄ (Docosahexaensäure) durch Expression von Fettsäureelongasen und/oder -desaturasen oder Produktion von Proteinen mit verbessertem Nahrungswert wie zum Beispiel mit einem hohen Anteil an essentiellen Aminosäuren (z.B. das methioninreiche 2S Albumingen der Brasilnuss). Bevorzugt sind Nukleinsäuren, die für das methioninreiche 2S Albumin aus Bertholletia excelsa (GenBank Acc.-No.:AB044391), die Δ6-Acyllipiddesaturase aus Physcomitrella patens (GenBank Acc.-No.: AJ222980; Girke et al. (1998) Plant J 15:39-48), die Δ6-Desaturase aus Mortierella alpina (Sakuradani et al. (1999) Gene 238:445-453), die Δ5-Desaturase aus Caenorhabditis elegans (Michaelson et al. 1998, FEBS Letters 439:215-218), die Δ5-Fettsäuredesaturase (des-5) aus Caenorhabditis elegans (GenBank Acc.-No.: AF078796), die Δ5-Desaturase aus Mortierella alpina (Michaelson et al. J Biol Chem 273:19055-19059), die Δ6-Elongase aus Caenorhabditis elegans (Beaudoin et al. (2000) Proc Natl. Acad Sci USA 97:6421-6426), die Δ6-Elongase aus Physcomitrella patens (Zank et al. (2000) Biochemical Society Transactions 28:654-657) oder funktionelle Äquivalente derselben kodieren.
7. Produktion von Feinchemikalien (wie beispielsweise Enzymen) und Pharmazeutika (wie zum Beispiel Antikörpern oder Vakzinen wie beschrieben bei Hood EE, Jilka JM. (1999) Curr Opin Biotechnol. 10(4):382-6; Ma JK, Vine ND (1999) Curr Top Microbiol Immunol 236:275-92). Beispielsweise konnte rekombinantes Avidin aus Hühnereiweiß und bakterieller β-Glucuronidase (GUS) in großen Maßstab in transgenen Maispflanzen produziert werden (Hood et al. (1999) Adv Exp Med Biol 464:127-47). Diese rekombinanten Proteine aus Maispflanzen werden von der Firma Sigma Chemicals Co. als hochreine Biochemikalien vertrieben.
8. Erreichen einer erhöhten Speicherfähigkeit in Zellen, die normalerweise weniger Speicherproteine oder -lipide enthalten mit dem Ziel, den Ertrag an diesen Substanzen zu erhöhen, zum Beispiel durch Expression eine Acetyl-CoA Carboxylase. Bevorzugt sind Nukleinsäuren, die für die Acetyl-CoA Carboxylase (Accase) aus Medicago sativa (GenBank Acc.-No.: L25042) oder funktionelle Äquivalente derselben kodieren.

Weitere Beispiele für vorteilhafte Gene sind zum Beispiel genannt bei Dunwell JM (2000) J Exp Bot. 51 Spec No:487-96.

Ferner können funktionelle Analoga der genannten Nukleinsäuren bzw. Proteine exprimiert werden. Funktionelle Analoga meint hier all die Sequenzen, die im wesentlichen die gleiche Funktion haben d.h. zu der Funktion (zum Beispiel einer Substratumsetzung oder einer Signaltransduktion) befähigt sind wie auch das beispielhaft genannte Protein. Dabei kann das funktionelle Analogon sich in anderen Merkmalen durchaus unterscheiden. Es kann zum Beispiel eine höhere oder niedrigere Aktivität haben oder auch über weitere Funktionalitäten verfügen. Funktionelle Analoga meint ferner Sequenzen, die für Fusionsproteine bestehend aus einem der bevorzugten Proteine und anderen Proteinen zum Beispiel einem weiteren bevorzugten Protein oder aber auch einer Signalpeptidsequenz kodieren.

Die Expression der Nukleinsäuren unter Kontrolle der erfindungsgemäßen Promotoren ist in jedem gewünschten Zellkompartiment, wie z.B. dem Endomembransystem, der Vakuole und den Chloroplasten möglich. Durch Nutzung des sekretorischen Weges sind gewünschte Glykosylierungsreaktionen, besondere Faltungen u.ä. möglich. Auch die Sekretion des Zielproteins zur Zelloberfläche bzw. die Sezemierung ins Kulturmedium, beispielsweise bei Nutzung suspensionskultivierter Zellen oder Protoplasten ist möglich. Die dafür notwendigen Targetsequenzen können sowohl in einzelnen Vektorvariationen berücksichtigt werden als auch durch Verwendung einer geeigneten Klonierungsstrategie gemeinsam mit dem zu klonierenden Zielgen in den Vektor mit eingebracht werden. Als Targetsequenzen können sowohl gen-eigene, sofern vorhanden, oder heterologe Sequenzen genutzt werden. Zusätzliche, heterologe zur funktionellen Verknüpfung bevorzugte aber nicht darauf beschränkte Sequenzen sind weitere Targeting-Sequenzen zur Gewährleistung der subzellulären Lokalisation im Apoplasten, in der Vakuole, in Plastiden, im Mitochondrium, im endoplasmatischen Retikulum (ER), im Zellkern, in Ölkörperchen oder anderen Kompartimenten; sowie Translationsverstärker wie die 5'-Leadersequenz aus dem Tabak-Mosaik-Virus (Gallie et al. (1987) Nucl Acids Res 15 8693-8711) und dergleichen. Das Verfahren, an sich nicht in den Plastiden lokalisierte Proteine, gezielt in die Plastiden zu transportieren, ist beschrieben (Klosgen RB & Weil JH (1991) Mol Gen Genet 225(2):297-304; Van Breusegem F et al. (1998) Plant Mol Biol 38(3):491-496). Bevorzugte Sequenzen sind
a) kleine Untereinheit (SSU) der Ribulosebisphosphatcarboxylase (Rubisco ssu) aus Erbse, Mais, Sonnenblume
b) Transitpeptide abgeleitet von Genen der pflanzlichen Fettsäurebiosynthese wie das Transitpeptid des plastidären "Acyl Carrier Protein" (ACP), die Stearyl-ACP-Desaturase, β-Ketoacyl-ACP Synthase oder die Acyl-ACP-Thioesterase
c) das Transitpeptid für GBSSI ("Starch Granule Bound Starch Synthase I")
d) LHCP II Gene.

Die Zielsequenzen können mit anderen, von dem Transitpeptid kodierenden Sequenzen verschiedenen, Targeting-Sequenzen verknüpft sein, um eine subzellulären Lokalisation im Apoplasten, in der Vakuole, in Plastiden, im Mitochondrium, im Endoplasmatischen Retikulum (ER), im Zellkern, in Ölkörperchen oder anderen Kompartimenten zu gewährleisten. Ferner können Translationsverstärker wie die 5'-Leadersequenz aus dem Tabak-Mosaik-Virus (Gallie et al. (1987) Nucl Acids Res 15:8693-8711) und dergleichen zum Einsatz kommen.

Dem Fachmann ist ferner bekannt, dass er die oben beschriebenen Gene nicht direkt unter Verwendung der für diese Gene kodierenden Nukleinsäuresequenzen exprimieren oder zum Beispiel durch anti-sense reprimieren muss. Er kann auch zum Beispiel künstliche Transkriptionsfaktoren vom Typ der Zinkfingerproteine verwenden (Beerli RR et al. (2000) Proc Natl Acad Sci USA 97(4):1495-500). Diese Faktoren lagern sich in den regulatorischen Bereichen der zu exprimierenden oder zu reprimierenden endogenen Gene an und bewirken, je nach Gestaltung des Faktors, eine Expression oder Repression des endogenen Gens. So kann man die gewünschten Effekte auch durch Expression eines entsprechenden Zinkfinger-Transkriptionsfaktors unter Kontrolle eines der erfindungsgemäßen Promotoren erreichen.

Die erfindungsgemäßen Expressionskassetten können ebenso zur Unterdrückung bzw. Reduktion von Replikation oder/und Translation von Zielgenen durch "Gene Silencing" eingesetzt werden.

Die erfindungsgemäßen Expressionskassetten können auch eingesetzt werden, um Nukleinsäuren zu exprimieren, die sogenannte "antisense" Effekte vermitteln und so beispielsweise zur Verminderung der Expression eines Zielproteins befähigt sind.

Bevorzugte Gene bzw. Proteine, deren Suppression einen vorteilhaften Phänotyp bedingt, umfassen beispielhaft, aber nicht einschränkend:
a) Polygalakturonase zur Verhinderung von Zellabbau und "Matschig"-werden von Pflanzen und Früchten beispielsweise Tomaten. Bevorzugt werden dazu Nukleinsäuresequenzen wie die des Polygalakturonase-Gens der Tomate (GenBank Acc.-No.: X14074) oder dessen Homologe aus anderen Gattungen und Arten verwendet.
b) Verminderung der Expression von allergenen Proteinen wie beispielsweise beschrieben bei Tada Y et al. (1996) FEBS Lett 391(3):341-345 oder Nakamura R (1996) Biosci Biotechnol Biochem 60(8):1215-1221.
c) Veränderung der Blütenfarbe durch Suppression der Expression von Enzymen der Anthocyanbiosynthese. Entsprechende Vorgehensweisen sind beschrieben (beispielsweise bei Forkmann G, Martens S. (2001) Curr Opin Biotechnol 12(2):155-160). Bevorzugt werden dazu Nukleinsäuresequenzen wie die der Ftavonoid-3'-hydroxylase (GenBank Acc.-No.: AB045593), der Dihydroflavanol-4-reduktase (GenBank Acc.-No.: AF017451), der Chalconisomerase (GenBank Acc.-No.: AF276302), der Chalconsynthase (GenBank Acc.-No.: AB061022), der Flavanone-3-beta-hydroxylase (GenBank Acc.-No.: X72592) oder der Flavonesynthase II (GenBank Acc.-No.: AB045592) oder deren Homologe aus anderen Gattungen und Arten verwendet.
d) Verschiebung des Amylose/Amylopektingehaltes in Stärke durch Suppression des Verzweigungsenzyms Q, das für die α-1,6-glykosidische Verknüpfung verantwortlich ist. Entsprechende Vorgehensweisen sind beschrieben (beispielsweise bei Schwall GP et al. (2000) Nat Biotechnol 18(5):551-554). Bevorzugt werden dazu Nukleinsäuresequenzen wie die des Starch branching enzyme II der Kartoffel (GenBank Acc.-No.: AR123356; US 6,169,226) oder dessen Homologe aus anderen Gattungen und Arten verwendet.

Eine "antisense" Nukleinsäure meint zunächst eine Nukleinsäuresequenz die ganz oder teilweise zu zumindest einem Teil des "sense"-Stranges besagten Zielproteins komplementär ist. Dem Fachmann ist bekannt, dass er alternativ die cDNA oder das korrespondierendes Gen als Ausgangsmatrize für entsprechende antisense-Konstrukte verwenden kann. Bevorzugt ist die "antisense" Nukleinsäure komplementär zu dem kodierenden Bereich des Zielproteins oder einem Teil desselben. Die "antisense" Nukleinsäure kann aber auch zu der nicht-kodierenden Region oder einem Teil derselben komplementär sein. Ausgehend von der Sequenzinformation zu einem Zielprotein, kann eine antisense Nukleinsäure unter Berücksichtigung der Basenpaarregeln von Watson und Crick in der dem Fachmann geläufigen Weise entworfen werden. Eine antisense Nukleinsäure kann komplementär zu der gesamten oder einem Teil der Nukleinsäuresequenz eines Zielproteins sein. In einer bevorzugten Ausführungsform ist die antisense Nukleinsäure ein Oligonukleotid mit einer Länge von zum Beispiel 15, 20, 25, 30, 35, 40, 45 oder 50 Nukleotiden.

Die antisense Nukleinsäure umfasst in einer bevorzugten Ausführungsform α-anomere Nukleinsäuremoleküle. α-Anomere Nukleinsäuremoleküle bilden besondere doppelsträngige Hybride mit komplementärer RNA in denen im Unterschied zu den normalen β-Einheiten die Stränge parallel zu einander verlaufen (Gaultier et al. (1987) Nucleic Acids Res 15:6625-6641).

Ebenso umfasst ist die Verwendung der oben beschriebenen Sequenzen in sense-Orientierung, was wie dem Fachmann geläufig ist, zu einer Kosuppression führen kann. Die Expression von sense-RNA zu einem endogenen Gen kann dessen Expression vermindern oder ausschalten, ähnlich wie es für antisense Ansätze beschrieben wurde (Goring et al. (1991) Proc Natl Acad Sci USA 88:1770-1774; Smith et al. (1990) Mol Gen Genet 224:447-481; Napoli et al. (1990) Plant Cell 2:279-289; Van der Krol et al. (1990) Plant Cell 2:291-299). Dabei kann das eingeführte Konstrukt das zu vermindernde Gen ganz oder nur teilweise repräsentieren. Die Möglichkeit zur Translation ist nicht erforderlich.

Ganz besonders bevorzugt ist auch die Verwendung von Verfahren wie der Genregulation mittels doppelsträngiger RNA ("double-stranded RNA interference"). Entsprechende Verfahren sind dem Fachmann bekannt und im Detail beschrieben (z.B. Matzke MA et al. (2000) Plant Mol Biol 43:401-415; Fire A. et al (1998) Nature 391:806-811; WO 99/32619; WO 99/53050; WO 00/68374; WO 00/44914; WO 00/44895; WO 00/49035; WO 00/63364). Auf die in den angegebenen Zitaten beschriebenen Verfahren und Methoden wird ausdrücklich Bezug genommen. Hier wird durch gleichzeitige Einbringung von Strang- und Gegenstrang eine hocheffiziente Unterdrückung nativer Gene bewirkt.

Vorteilhaft kann die antisense-Strategie mit einem Ribozym-Verfahren gekoppelt werden. Ribozyme sind katalytisch aktive RNA Sequenzen, die gekoppelt an die antisense Sequenzen, die Zielsequenzen katalytisch spalten (Tanner NK. FEMS Microbiol Rev. 1999; 23 (3):257-75). Dies kann die Effizienz einer anti-sense Strategie erhöhen. Die Expression von Ribozymen zur Verminderung bestimmter Proteine ist dem Fachmann bekannt und beispielsweise beschrieben in EP-A1 0 291 533, EP-A1 0 321 201 und EP-A1 0 360 257. Geeignete Zielsequenzen und Ribozyme können zum Beispiel wie bei Steinecke (Ribozymes, Methods in Cell Biology 50, Galbraith et al. eds. Academic Press, Inc. (1995), 449-460) beschrieben, durch Sekundärstrukturberechnungen von Ribozym- und Ziel-RNA sowie durch deren Interaktion bestimmt werden (Bayley CC et al., Plant Mol Biol. 1992; 18(2):353-361; Lloyd AM and Davis RW et al., Mol Gen Genet. 1994 Mar;242(6):653-657). Beispielhaft sind "hammerhead"-Ribozyme zu nennen (Haselhoff and Gerlach (1988) Nature 334:585-591). Bevorzugte Ribozyme basieren auf Derivaten der Tetrahymena L-19 IVS RNA (US 4,987,071; US 5,116,742). Weitere Ribozyme mit Selektivität für eine L119 mRNA können selektioniert werden (Bartel D und Szostak JW (1993) Science 261:1411-1418).

In einer weiteren Ausführungsform kann Verminderung der Zielprotein-Expression unter Verwendung von Nukleinsäuresequenzen bewirkt werden, die komplementär zu regulativen Elementen der Zielprotein-Gene sind, mit diesen eine triple-helikale Struktur ausbilden und so die Gen-Transkription verhindern (Helene C (1991) Anticancer Drug Des. 6(6):569-84; Helene C et al. (1992) Ann NY Acad Sci 660:27-36; Mäher LJ (1992) Bioassays 14(12):807-815).

Die erfindungsgemäßen bidirektionalen Promotoren sind besonders vorteilhaft, wenn er für die Regulation zweier Enzyme eines Stoffwechselweges eingesetzt wird. Beispielsweise kann die 2'-Methyl-6-phytylhydroquinon-methyltransferase und die Homogentisatphytylpyrophosphattransferase gleichzeitig über einen der erfindungsgemäßen bidirektionalen Promoter exprimiert werden, was einen Anstieg von Tocopherolen bewirkt. Weiterhin führt die Hemmung der Homogentisatdioxygenase (beispielsweise über die Expression einer korrespondierenden dsRNA) und die Überexpression der Tyrosinaminotransferase zu einer Steigerung des Tocopherolgehaltes. Im Carotinoidstoffwechsel führt die Hemmung der ε-Zyklase und die Überexpression der β-Zyklase zu einer Veränderung des Gehaltes von α-Carotin und β-Carotin.

Es ist möglich postranskriptionellen "Silencing"-Effekte durch parallele Hemmung der Transkription des SDE3 Gens und Überexpression des rekombinanten Proteins zu verhindern (WO 02/063039).

Auch immunologisch aktive Teilen von Antikörpern können unter Verwendung der erfindungsgemäßen Promotoren vorteilhaft exprimiert werden. So kann beispielsweise in die eine Richtung die schwere Kette eines IgG1 Antikörpers und in die andere Richtung die leichte Kette exprimiert werden. Nach Translation bilden beide einen funktionellen Antikörper (WO 02/101006).

Weiterhin können gleichzeitig stressbezogene Ionentransporter (WO 03/057899) zusammmen mit Herbizidgenen exprimiert werden, um die Toleranz gegen Umwelteinflüsse zu erhöhen.

Viele Enzyme bestehen aus zwei oder mehreren Untereinheiten, die beide notwendig für die Funktion sind. Mittels eines der erfindungsgemäßen, bidirektionalen Promotoren ist es möglich, zwei Untereinheiten gleichzeitig zu exprimieren. Ein Beispiel dafür ist die Überexpression der α- und der β- Untereinheit des Follikel stimulierendes menschlichen Hormons.

Für die Etablierung von Transformationssystemen ist ein Konstrukt bestehend aus einem Gen für eine Selektionsmarker und einem Reportergen besonders wertvoll, wenn sie durch diesen bidirektionalen Promotor reguliert werden.

Die erfindungsgemäßen Expressionskassetten und die von ihnen abgeleiteten Vektoren können weitere Funktionselemente enthalten. Der Begriff Funktionselement ist breit zu verstehen und meint all solche Elemente, die einen Einfluss auf Herstellung, Vermehrung oder Funktion der erfindungsgemäßen Expressionskassetten oder von diesen abgeleitete Vektoren oder Organismen haben. Beispielhaft aber nicht einschränkend seien zu nennen:
a) Reportergene
   Reportergene oder -proteine kodieren für leicht quantifizierbare Proteine und gewährleisten über Eigenfarbe oder Enzymaktivität eine Bewertung der Transformationseffizienz, des Expressionsortes oder -zeitpunktes (Schenbom E, Groskreutz D (1999) Mol Biotechnol 13(1):29-44). Beispielhaft sind zu nennen:
   - "green fluorescence protein" (GFP) (Chui WL et al., Curr Biol 1996, 6:325-330; Leffel SM et al., Biotechniques. 23(5):912-8, 1997; Sheen et al.(1995) Plant Journal 8(5):777-784; Haseloff et al.(1997) Proc Natl Acad Sci USA 94(6):2122-2127; Reichel et al.(1996) Proc Natl Acad Sci USA 93(12):5888-5893; Tian et al. (1997) Plant Cell Rep 16:267-271; WO 97/41228).
   - Chloramphenicoltransferase (Fromm et al. (1985) Proc Natl Acad Sci USA 82:5824-5828),
   - Luziferase (Millar et al. (1992) Plant Mol Biol Rep 10:324-414; Ow et al. (1986) Science, 234:856-859); erlaubt Bioluminescenzdetektion.
   - β-Galactosidase, kodiert für ein Enzym für das verschiedenen chromogene Substrate zur Verfügung stehen.
   - β-Glucuronidase (GUS) (Jefferson et al. (1987) EMBO J 6:3901-3907) oder das uidA Gen, das ein Enzym für verschiedene chromogene Substrate kodiert.
   - R-Locus Genprodukt:Protein, das die Produktion von Anthocyaninpigmenten (rote Färbung) in pflanzlichen Gewebe reguliert und so eine direkte Analyse der Promotoraktivität ohne Zugabe zusätzlicher Hilfsstoffe oder chromogener Substrate ermöglicht (Dellaporta et al., In: Chromosome Structure and Function: Impact of New Concepts, 18th Stadler Genetics Symposium 11:263-282, 1988).
   - β-Lactamase (Sutcliffe (1978) Proc Natl Acad Sci USA 75:3737-3741), Enzym für verschiedene chromogene Substrate (z.B. PADAC, ein chromogenes Cephalosporin).
   - xylE Genprodukt (Zukowsky et al. (1983) Proc Natl Acad Sci USA 80:1101-1105), Catecholdioxygenase, die chromogene Catechole umsetzen kann.
   - Alpha-Amylase (Ikuta et al. (1990) Bio/Technol. 8:241-242).
   - Tyrosinase (Katz et al.(1983) J Gen Microbiol 129:2703-2714), Enzym, das Tyrosin zu DOPA und Dopaquinon oxidiert, die infolge das leicht nachweisbare Melanin bilden.
   - Aequorin (Prasher et al.(1985) Biochem Biophys Res Commun 126(3):1259-1268), kann in der Calcium-sensitiven Bioluminescenzdetektion verwendet werden.
b) Replikationsursprünge, die eine Vermehrung der erfindungsgemäßen Expressionskassetten oder Vektoren in zum Beispiel E. coli gewährleisten. Beispielhaft seien genannt ORI (origin of DNA replication), der pBR322 ori oder der P15A ori (Sambrook et al.: Molecular Cloning. A Laboratory Manual, 2nd ed. Cold Spring Harbor Laboratory Press, Cold Spring Harbor, NY, 1989).
c) Elemente zum Beispiel "Bordersequenzen", die einen Agrobakterien-vermittelten Transfer in Pflanzenzellen für die Übertragung und Integration ins Pflanzengenom ermöglichen, wie zum Beispiel die rechte oder linke Begrenzung der T-DNA oder die vir-Region.
d) Multiple Klonierungsregionen (MCS) erlauben und erleichtern die Insertion eines oder mehrerer Nukleinsäuresequenzen.

Dem Fachmann sind verschiedene Wege bekannt, um zu einer erfindungsgemäßen Expressionskassette zu gelangen. Die Herstellung einer erfindungsgemäßen Expressionskassette erfolgt beispielsweise durch Fusion eines der erfindungsgemäßen Promotoren (oder eines funktionellen Äquivalentes oder funktionell äquivalenten Teils gemäß SEQ ID NO: 1 oder 2 oder eines funktionellen Äquivalentes mit einer zu exprimierenden Nukleinsäuresequenz, gegebenenfalls einer für eine Transitpeptid kodierenden Sequenz, vorzugsweise ein chloroplastenspezifisches Transitpeptid, welches vorzugsweise zwischen dem Promotor und der jeweiligen Nukleinsäuresequenz angeordnet ist, sowie einem Terminator- oder Polyadenylierungssignal. Dazu verwendet man gängige Rekombinations- und Klonierungstechniken (wie oben beschrieben).

Unter einer Expressionskassette sind aber auch solche Konstruktionen zu verstehen, bei denen der Promotor, ohne dass er zuvor mit einer zu exprimierenden Nukleinsäuresequenz funktionell verknüpft wurde, zum Beispiel über eine gezielte homologe Rekombination oder eine zufällige Insertion in ein Wirtsgenom eingeführt wird, dort regulatorische Kontrolle über mit ihm dann funktionell verknüpfte Nukleinsäuresequenzen übernimmt und die transgene Expression derselben steuert. Durch Insertion des Promotors - zum Beispiel durch eine homologe Rekombination - vor eine für ein bestimmtes Polypeptid kodierende Nukleinsäure erhält man eine erfindungsgemäße Expressionskassette, welche die Expression des bestimmten Polypeptides in der Pflanze steuert. Ferner kann die Insertion des Promotors auch derart erfolgen, dass antisense-RNA zu der für ein bestimmtes Polypeptid kodierenden Nukleinsäure exprimiert wird. Damit wird die Expression des bestimmten Polypeptides in Pflanzen herunterreguliert oder ausgeschaltet.

Analog kann auch eine transgen zu exprimierende Nukleinsäuresequenz zum Beispiel durch eine homologe Rekombination hinter den endogenen, natürlichen Promotor platziert werden, wodurch man eine erfindungsgemäße Expressionskassette erhält, welche die Expression der transgen zu exprimierenden Nukleinsäuresequenz steuert.

Erfindungsgemäß sind ferner Vektoren, die die oben beschriebenen Expressionskassetten enthalten. Vektoren können beispielhaft Plasmide, Cosmide, Phagen, Viren oder auch Agrobakterien sein.

Ein anderer Gegenstand der Erfindung betrifft transgene Organismen, transformiert mit wenigstens einer erfindungsgemäßen Expressionskassette oder einem erfindungsgemäßen Vektor, sowie Zellen, Zellkulturen, Gewebe, Teile - wie zum Beispiel bei pflanzlichen Organismen Blätter, Wurzeln usw. - oder Vermehrungsgut abgeleitet von solchen Organismen.

Unter Organismus, Ausgangs- oder Wirtsorganismen werden Organismen verstanden.

Eingeschlossen sind im Rahmen der Erfindung alle Gattungen und Arten höherer und niedrigerer Pflanzen des Pflanzenreiches. Eingeschlossen sind ferner die reifen Pflanzen, Saatgut, Sprossen und Keimlinge, sowie davon abgeleitete Teile, Vermehrungsgut und Kulturen, zum Beispiel Zellkulturen. Reife Pflanzen meint Pflanzen zu jedem beliebigen Entwicklungsstadium jenseits des Keimlings. Keimling meint eine junge, unreife Pflanze in einem frühen Entwicklungsstadium.

Einjährige, mehrjährige, monocotyledone und dicotyledone Pflanzen sind bevorzugte Wirtsorganismen für die Herstellung transgener Pflanzen. Bevorzugt sind Pflanzen nachfolgender Pflanzenfamilien: Amaranthaceae, Asteraceae, Brassicaceae, Carophyllaceae, Chenopodiaceae, Compositae, Cruciferae, Cucurbitaceae, Labiatae, Leguminosae, Papilionoideae, Liliaceae, Linaceae, Malvaceae, Rosaceae, Rubiaceae, Saxifragaceae, Scrophulariaceae, Solanacea, Sterculiaceae, Tetragoniacea, Theaceae, Umbelliferae.

Bevorzugte monokotyle Pflanzen sind insbesondere ausgewählt aus den monokotylen Kulturpflanzen, wie zum Beispiel der Familie der Gramineae wie Reis, Mais, Weizen oder andere Getreidearten wie Gerste, Hirse, Roggen, Triticale oder Hafer sowie dem Zuckerrohr sowie alle Arten von Gräsern.

Bevorzugte dikotyle Pflanzen sind insbesondere ausgewählt aus den dikotylen Kulturpflanzen, wie zum Beispiel
- Asteraceae wie Sonnenblume, Tagetes oder Calendula und andere mehr,
- Compositae, besonders die Gattung Lactuca, ganz besonders die Art sativa (Salat) und andere mehr,
- Cruciferae, besonders die Gattung Brassica, ganz besonders die Arten napus (Raps), campestris (Rübe), oleracea cv Tastie (Kohl), oleracea cv Snowball Y (Blumenkohl) und oleracea cv Emperor (Broccoli) und weitere Kohlarten; und der Gattung Arabidopsis, ganz besonders die Art thaliana und andere mehr,
- Cucurbitaceae wie Melone, Kürbis oder Zucchini und andere mehr,
- Leguminosae besonders die Gattung Glycine, ganz besonders die Art max (Sojabohne) Soja sowie Alfalfa, Erbse, Bohnengewächsen oder Erdnuss und andere mehr
- Rubiaceae, bevorzugt der Unterklasse Lamiidae wie beispielsweise Coffea arabica oder Coffea liberica (Kaffestrauch) und andere mehr,
- Solanaceae besonders die Gattung Lycopersicon, ganz besonders die Art esculentum (Tomate) und die Gattung Solanum, ganz besonders die Art tuberosum (Kartoffel) und melongena (Aubergine) sowie Tabak oder Paprika und andere mehr,
- Sterculiaceae, bevorzugt der Unterklasse Dilleniidae wie beispielsweise Theobroma cacao (Kakaostrauch) und andere mehr,
- Theaceae, bevorzugt der Unterklasse Dilleniidae wie beispielsweise Camellia sinensis oder Thea sinensis (Teestrauch) und andere mehr,
- Umbelliferae, besonders die Gattung Daucus (ganz besonders die Art carota (Karotte) und Apium (ganz besonders die Art graveolens dulce (Selarie) und andere mehr; und die Gattung Capsicum, ganz besonders die Art annum (Pfeffer) und andere mehr,
sowie Lein, Soja, Baumwolle, Hanf (Flachs), Gurke, Spinat, Möhre, Zuckerrübe und den verschiedenen Baum-, Nuss- und Weinarten, insbesondere Banane und Kiwi.

Bevorzugt sind Nicotiana tabacum, Tagetes erecta und Calendula officinalis sowie alle Gattungen und Arten, die als Nahrungs- oder Futtermittel zum Einsatz kommen, wie die beschriebenen Getreidearten, oder sich zur Herstellung von Ölen eignen, wie Ölsaaten (wie Raps), Nussarten, Soja, Sonnenblume, Kürbis und Erdnuss.

Am meisten bevorzugt sind alle Pflanzen der Familie der Brassicaceae, ganz besonders die Brassica Arten wie Brassica napus (Raps), campestris (Rübe), oleracea cv Tastie (Kohl), oleracea cv Snowball Y (Blumenkohl) und oleracea cv Emperor (Broccoli) und weitere Kohlarten; sowie der Gattung Arabidopsis, ganz besonders die Art thaliana.

Pflanzliche Organismen im Sinne der Erfindung sind weiterhin weitere photosynthetisch aktive befähigte Organismen, wie zum Beispiel Algen oder Cyanobakterien, sowie Moose. Bevorzugte Algen sind Grünalgen, wie beispielsweise Algen der Gattung Haematococcus, Phaedactylum tricomatum, Volvox oder Dunaliella. Insbesondere bevorzugt sind, Algen wie Chlorophyceae, Phaeophpyceae, Rhodophyceae, Myxophyceae, Xanthophyceae, Bacillariophyceae (Diatomeen) und Euglenophyceae.

Die Herstellung eines transformierten Organismus oder einer transformierten Zelle erfordert, dass die entsprechende DNA in die entsprechende Wirtszelle eingebracht wird. Für diesen Vorgang, der als Transformation bezeichnet wird, steht eine Vielzahl von Methoden zur Verfügung (siehe auch Keown et al. (1990) Methods in Enzymology 185:527-537). So kann die DNA beispielhaft direkt durch Mikroinjektion oder durch Bombardierung mit DNA-beschichteten Mikropartikeln eingeführt werden. Auch kann die Zelle chemisch, zum Beispiel mit Polyethylenglycol, permeabilisiert werden, so dass die DNA durch Diffusion in die Zelle gelangen kann. Die DNA kann auch durch Protoplastenfusion mit anderen DNA-enthaltenden Einheiten wie Minicells, Zellen, Lysosomen oder Liposomen erfolgen. Elektroporation ist eine weitere geeignete Methode zur Einführung von DNA, bei der die Zellen reversibel durch einen elektrischen Impuls permeabilisert werden.

Bei Pflanzen werden dabei die beschriebenen Methoden zur Transformation und Regeneration von Pflanzen aus Pflanzengeweben oder Pflanzenzellen zur transienten oder stabilen Transformation genutzt. Geeignete Methoden sind vor allem die Protoplastentransformation durch Polyethylenglykol-induzierte DNA-Aufnahme, das biolistische Verfahren mit der Genkanone, die sogenannte particle bombardment Methode, die Elektroporation, die Inkubation trockener Embryonen in DNA-haltiger Lösung und die Mikroinjektion.

Neben diesen "direkten" Transformationstechniken kann eine Transformation auch durch bakterielle Infektion mittels Agrobacterium tumefaciens oder Agrobacterium rhizogenes durchgeführt werden. Diese Stämme enthalten ein Plasmid (Ti bzw. Ri Plasmid), das auf die Pflanze nach Agrobacterium-Infektion übertragen wird. Ein Teil dieses Plasmids, genannt T-DNA (transferred DNA), wird in das Genom der Pflanzenzelle integriert.

Die Agrobacterium-vermittelte Transformation ist am besten für dicotyledone, diploide Pflanzenzellen geeignet, wohingegen die direkten Transformationstechniken sich für jeden Zelltyp eignen.

Die Einführung einer erfindungsgemäßen Expressionskassette in pflanzliche Zellen kann vorteilhaft unter Verwendung von Vektoren realisiert werden.

In einer vorteilhaften Ausführungsform wird die Einführung der Expressionskassette mittels Plasmidvektoren realisiert. Bevorzugt sind solche Vektoren, die eine stabile Integration der Expressionskassette in das Wirtsgenom ermöglichen.

Im Falle von Injektion oder Elektroporation von DNA in pflanzliche Zellen sind keine besonderen Anforderungen an das verwendete Plasmid gestellt. Einfache Plasmide wie die der pUC-Reihe können verwendet werden. Sollen vollständige Pflanzen aus den transformierten Zellen regeneriert werden, so ist er erforderlich, das sich auf dem Plasmid ein zusätzliches selektionierbares Markergen befindet.

Transformationstechniken sind für verschiedene monokotyle und dikotyle pflanzliche Organismen beschrieben. Ferner stehen verschiedene mögliche Plasmidvektoren für die Einführung fremder Gene in Pflanzen zur Verfügung, die in der Regel einen Replikationsursprung für eine Vermehrung in E.coli und ein Markergen für eine Selektion transformierter Bakterien enthalten. Beispiele sind pBR322, pUC Reihe, M13mp Reihe, pACYC184 etc.

Die Expressionskassette kann in den Vektor über eine geeignete Restriktionsschnittstelle eingeführt werden. Das entstandene Plasmid wird zunächst in E.coli eingeführt. Korrekt transformierte E.coli werden selektioniert, gezüchtet und das rekombinante Plasmid mit dem Fachmann geläufigen Methoden gewonnen. Restriktionsanalyse und Sequenzierung können dazu dienen, den Klonierungsschritt zu überprüfen.

Transformierte Zellen d.h. solche, welche die eingeführte DNA integriert in die DNA der Wirtszelle enthalten, können von untransformierten selektioniert werden, wenn ein selektionierbarer Marker Bestandteil der eingeführten DNA ist. Als Marker kann beispielhaft jedes Gen fungieren, dass eine Resistenz gegen Antibiotika oder Herbizide zu verleihen vermag. Transformierte Zellen, die ein solches Markergen exprimieren, sind in der Lage, in der Gegenwart von Konzentrationen eines entsprechenden Antibiotikums oder Herbizides zu überleben, die einen untransformierten Wildtyp abtöten. Beispiel sind das bar Gen, dass Resistenz gegen das Herbizid Phosphinothricin verleiht (Rathore KS et al., Plant Mol Biol. 1993 Mar;21(5):871-884), das nptll Gen, dass Resistenz gegen Kanamycin verleiht, das hpt Gen, das Resistenz gegen Hygromycin verleiht, oder das EPSP-Gen, das Resistenz gegen das Herbizid Glyphosat verleiht.

Je nach Methode der DNA-Einführung können weitere Gene auf dem Vektorplasmid erforderlich sein. Werden Agrobacteria verwendet, so ist die Expressionskassette in spezielle Plasmide zu integrieren, entweder in einen Zwischenvektor (englisch: shuttle or intermediate vector) oder einen binären Vektor. Wenn zum Beispiel ein Ti oder Ri Plasmid zur Transformation verwendet werden soll, ist zumindest die rechte Begrenzung, meistens jedoch die rechte und die linke Begrenzung der Ti oder Ri Plasmid T-DNA als flankierende Region mit der einzuführenden Expressionskassette verbunden. Bevorzugt werden binäre Vektoren verwendet. Binäre Vektoren können sowohl in E.coli als auch in Agrobacterium replizieren. Sie enthalten in der Regel ein Selektionsmarkergen und einen Linker oder Polylinker flankiert von der rechten und linken T-DNA Begrenzungssequenz. Sie können direkt in Agrobacterium transformiert werden (Holsters et al. (1978) Mol. Gen. Genet. 163:181-187). Das Selektionsmarkergen erlaubt eine Selektion transformierter Agrobacteria und ist zum Beispiel das nptll Gen, das eine Resistenz gegen Kanamycin verleiht. Das in diesem Fall als Wirtsorganismus fungierende Agrobacterium sollte bereits ein Plasmid mit der vir-Region enthalten. Diese ist für die Übertragung der T-DNA auf die pflanzliche Zelle erforderlich. Ein so transformiertes Agrobacterium kann zur Transformation pflanzlicher Zellen verwendet werden.

Die Verwendung von T-DNA zur Transformation pflanzlicher Zellen ist intensiv untersucht und beschrieben (EP 120516; Hoekema, In: The Binary Plant Vector System, Offsetdrukkerij Kanters B.V., Alblasserdam, Chapter V; Fraley et al. (1986) CRC Crit. Rev. Plant. Sci., 4:1-46 and An et al. (1985) EMBO J. 4:277-287). Verschiedene binäre Vektoren sind bekannt und teilweise kommerziell erhältlich wie zum Beispiel pBIN19 (Clontech Laboratories, Inc. U.S.A.).

Für den Transfer der DNA in die pflanzliche Zelle werden pflanzliche Explantate mit Agrobacterium tumefaciens oder Agrobacterium rhizogenes kokultiviert. Ausgehend von infiziertem Pflanzenmaterial (z.B. Blatt-, Wurzel- oder Stengelteile, aber auch Protoplasten oder Suspensionen von Pflanzenzellen) können ganze Pflanzen unter Verwendung eines geeigneten Mediums, dass zum Beispiel Antibiotika oder Biozide zur Selektion transformierter Zellen enthalten kann, regeneriert werden. Die erhaltenen Pflanzen können dann auf die Präsenz der eingeführten DNA, hier der erfindungsgemäßen Expressionskassette, durchmustert werden. Sobald die DNA in das Wirtsgenom integriert ist, ist der entsprechende Genotyp in der Regel stabil und die entsprechende Insertion wird auch in den Nachfolgegenerationen wiedergefunden. In der Regel enthält die integrierte Expressionskassette einen Selektionsmarker (s.o.). Der Selektionsmarker erlaubt die Selektion von transformierten Zellen von untransformierten (McCormick et al. (1986) Plant Cell Rep 5:81-84). Die erhaltenen Pflanzen können in üblicher Weise gezüchtet und gekreuzt werden. Zwei oder mehr Generationen sollten kultiviert werden, um sicherzustellen, dass die genomische Integration stabil und vererblich ist.

Die genannten Verfahren sind beispielsweise in B. Jenes et al., Techniques for Gene Transfer, in: Transgenic Plants, Vol. 1, Engineering and Utilization, herausgegeben von Kung SD & Wu R, Academic Press (1993), S.128 - 143 sowie in Potrykus I (1991) Annu Rev Plant Physiol Plant Mol Biol 42:205-225) beschrieben. Vorzugsweise wird das zu exprimierende Konstrukt in einen Vektor kloniert, der geeignet ist, Agrobakterium tumefaciens zu transformieren, beispielsweise pBin19 (Bevan et al. (1984) Nucl Acids Res 12:8711f.).

Sobald eine transformierte Pflanzenzelle hergestellt wurde, kann eine vollständige Pflanze unter Verwendung von dem Fachmann bekannten Verfahren erhalten werden. Hierbei geht man beispielhaft von Kalluskulturen aus. Aus diesen noch undifferenzierten Zellmassen kann die Bildung von Spross und Wurzel in bekannter Weise induziert werden. Die erhaltenen Sprösslinge können ausgepflanzt und gezüchtet werden.

Die Wirksamkeit der Expression der transgen exprimierten Nukleinsäuren kann beispielsweise *in vitro* durch Sprossmeristemvermehrung unter Verwendung einer der oben beschriebenen Selektionsmethoden ermittelt werden.

Erfindungsgemäß sind ferner von den oben beschriebenen transgenen Organismen abgeleitete Zellen, Zellkulturen, Teile - wie zum Beispiel bei transgenen pflanzlichen Organismen Wurzeln, Blätter etc.-, und transgenes Vermehrungsgut wie Saaten oder Früchte.

Von Menschen und Tieren verzehrbare erfindungsgemäße, genetisch veränderte Pflanzen können auch beispielsweise direkt oder nach an sich bekannter Aufbereitung als Nahrungsmittel oder Futtermittel verwendet werden.

Ein weiterer Gegenstand der Erfindung betrifft die Verwendung der oben beschriebenen erfindungsgemäßen, transgenen Organismen und der von ihnen abgeleitete Zellen, Zellkulturen, Teile - wie zum Beispiel bei transgenen pflanzlichen Organismen Wurzeln, Blätter etc.-, und transgenes Vermehrungsgut wie Saaten oder Früchte, zur Herstellung von Nahrungs- oder Futtermitteln, Pharmazeutika oder Feinchemikalien.

Bevorzugt ist ferner ein Verfahren zur rekombinanten Herstellung von Pharmazeutika oder Feinchemikalien in Wirtsorganismen wobei ein Wirtsorganismus mit einer der oben beschriebenen Expressionskassetten oder Vektoren transformiert wird und diese Expressionskassette ein oder mehrere Strukturgene enthält, die für die gewünschte Feinchemikalie kodieren oder die Biosynthese der gewünschten Feinchemikalie katalysieren, der transformierte Wirtsorganismus gezüchtet wird und die gewünschte Feinchemikalie aus dem Züchtungsmedium isoliert wird. Dieses Verfahren ist für Feinchemikalien wie Enzyme, Vitamine, Aminosäuren, Zucker, Fettsäuren, natürliche und synthetische Geschmacks-, Aroma- und Farbstoffe breit anwendbar. Besonders bevorzugt ist die Produktion von Tocopherolen und Tocotrienolen sowie Carotinoiden. Die Züchtung der transformierten Wirtsorganismen sowie die Isolierung aus den Wirtsorganismen bzw. aus dem Züchtungsmedium erfolgt mittels der dem Fachmann bekannten Verfahren. Die Produktion von Pharmazeutika, wie zum Beispiel Antikörpern oder Vakkzinen ist beschrieben bei Hood EE, Jilka JM (1999). Curr Opin Biotechnol 10(4):382-6; Ma JK, Vine ND (1999). Curr Top Microbiol Immunol 236:275-92.

### Sequenzen

| | |
|---|---|
| 1. SEQ ID NO: 1 | Bidirektionaler Promotor aus Arabidopsis thaliana. Intergenische Region zwischen dem putativen FD-Gen und dem putativem OASTL Gen bis jeweils vor die angenommenen Transkriptionsstarts. |
| | |
| 2. SEQ ID NO: 2 | Bidirektionaler Promotor aus Arabidopsis thaliana einschließlich der 5'-untranslatierten Regionen des putativen FD-Gen und des putativen OASTL Gen bis jeweils vor die ATG-Start-Codons. Im Vergleich zu der nativen Sequenz umfasst vorliegende Sequenz ein zusätzliches C an Position 4 gegenüber der nativen Arabidopsissequenz durch die Einführung einer BamHl Erkennungssequenz. |
| | |
| 3. SEC ID NO: 3 | Sequenz des Plasmids pUH200. Das GUS Gen wird in Richtung des FD Gens exprimiert, das nptll-Gen in Richtung des OASTL Gens. |
| | |
| 4. SEC ID NO: 4 | Sequenz des Plasmids pUH201. Das GUS Gen wird in Richtung des OASTL Gens exprimiert, das nptll-Gen in Richtung des FD Gens. |
| | |
| 5. SEC ID NO: 5 | Oligonukleotid-Primer pFD3 |
| | 5'-**acggatcc**gagagacagagagacggagacaaaa-3' |
| | |
| 6. SEQ ID NO: 6 | Oligonukleotid-Primer pFD4 5'-gcggatccaagcttcactgcttaaattc-3' |

### Beschreibung der Abbildungen

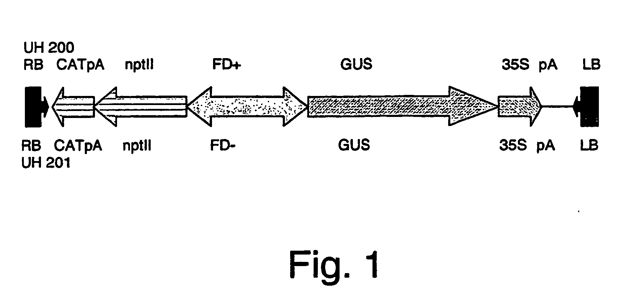

- Fig. 1:: Schematische Darstellung der bidirektionalen Einheit in den Vektoren UH200 und UH201. RB: Rechte Grenze ("Border") der Agrobacterium T-DNA; CATpA: Terminators des Cathepsin D Inhibitor; nptll: Neomycinphosphotransferase II Gen (Kanamycin-Resistenz-Gen); FD: Intergenische Region zwischen FD und OASTL Gen (+/- geben die Leserichtung des FD-Gens an); GUS: β-Glucuronidase-Gen; 35SpA: Terminator des 35S CaMV Gens; LB: Linke Grenze ("Border") der Agrobacterium T-DNA.

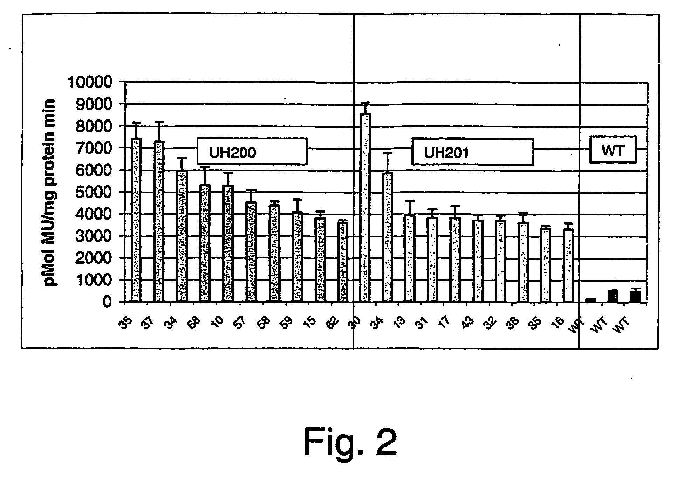

- Fig. 2:: Analyse der GUS Aktivität in Blättern transgener Rapspflanzen transformiert mit UH 200 (Orientierung des Ferredoxingens) bzw. UH 201 (Orientierung des OASTL Gens) im Vergleich zu Wildtyp (WT) Pflanzen. Gezeigt sind die Ergebnisse verschiedener Linien von UH200 bzw UH201 transformierter Raps-Pflanzen (gekennzeichnet durch Nummer der jeweiligen-Linie auf der x-Achse). Die GUS-Aktivität ist pMol Methylumbelliferon (MU) / mg (Protein) min angegeben.

### Beispiele

### Allgemeine Methoden:

Die chemische Synthese von Oligonukleotiden kann beispielsweise, in bekannter Weise, nach der Phosphoamiditmethode (Voet, Voet, 2. Auflage, Wiley Press New York, Seite 896-897) erfolgen. Die im Rahmen der vorliegenden Erfindung durchgeführten Klonierungsschritte wie z.B. Restriktionsspaltungen, Agarosegelelektrophoresen, Reinigung von DNA-Fragmenten, Transfer von Nukleinsäuren auf Nitrozellulose und Nylonmembranen, Verknüpfen von DNA-Fragmenten, Transformation von E. coli Zellen, Anzucht von Bakterien, Vermehrung von Phagen und Sequenzanalyse rekombinanter DNA werden wie bei Sambrook et al. (1989) Cold Spring Harbor Laboratory Press; ISBN 0-87969-309-6 beschrieben durchgeführt. Die Sequenzierung rekombinanter DNA-Moleküle erfolgt mit einem Laserfluoreszenz-DNA-Sequenzierer der Firma ABI nach der Methode von Sanger (Sanger et al. (1977) Proc Natl Acad Sci USA 74:5463-5467).

### Beispiel 1: Isolierung von genomischer DNA aus Arabidopsis thaliana, Tabak und Raps

Die genomische DNA aus *Arabidopsis thaliana,* Tabak und Raps wurde mithilfe des DNeasy Plant Mini Kit von Qiagen Kat. No. 60106 entsprechend der Vorschrift isoliert.

### Beispiel 2: Transformation von Tabak und Raps

Die Transformation von Tabak erfolgte über Infektion mit *Agrobacterium tumefaciens* gemäß der von Horsch entwickelten Methode (Horsch et al. (1985) Science 227: 1229-1231). Alle zur Transformation verwendeten Konstrukte wurden anhand der Gefrier/Tau-Methode (wiederholtes Auftauen und Einfrieren) in Agrobacterium tumefaciens transformiert. Die das gewünschte Konstrukt enthaltenden *Agrobacterium*-Kolonien wurden auf YEB Medium (1 % Rinderextrakt (Difco), 0,5% Caseinenzym-hydrolysat, 0,1% Hefeextrakt(Duchefa), 0,5% Saccharose, 2 mM MgSO₄, 1,5% Agar) -Medium mit 50 µg/ml Kanamycin, 40 µg/ml Gentamycin, 100 µg/ml Spectinomycin und 25 µg/ml Rifampicin selektioniert.

Zur Transformation von Tabakpflanzen (*Nicotiana tabacum* L. cv. *Samsun* NN) wurden 10 ml einer unter Selektion gewachsenen Übernachtkultur von *Agrobacterium tumefaciens* abzentrifugiert, der Überstand verworfen, und die Bakterien im gleichen Volumen Antibiotika-freien Mediums resuspendiert. In einer sterilen Petrischale wurden Blattscheiben steriler Pflanzen (Durchmesser ca. 1 cm) in dieser Bakterienlösung gebadet. Anschließend wurden die Blattscheiben in Petrischalen auf MS-Medium (Murashige und Skoog (1962) Physiol Plant 15:473ff.) mit 2% Saccharose und 0.8% Bacto-Agar ausgelegt. Nach 2-tägiger Inkubation im Dunkeln bei 25°C wurden sie auf MS-Medium mit 100 mg/l Kanamycin, 500 mg/l Claforan, 1 mg/l Benzylaminopurin (BAP), 0,2 mg/l Naphtylessigsäure (NAA), 1,6% Glukose und 0,8% Bacto-Agar übertragen und die Kultivierung (16 Stunden Licht / 8 Stunden Dunkelheit) fortgesetzt. Wachsende Sprosse wurden auf hormonfreies MS-Medium mit 2% Saccharose, 250 mg/l Claforan und 0,8% Bacto-Agar überführt.

Die Transformation von Raps erfolgte mittels der Petiolentransformation nach Moloney et al. (Moloney MM et al. (1989) Plant Cell Rep 8:238-242).

### Beispiel 3: Untersuchung zur bidirektionalen Expression des FD Promoters

### a) PCR Isolierung des FD Promoters aus Arabidopsis thaliana

Der putative bidirektionale Promoter wurde mittels PCR aus genomischer *Arabidopsis thaliana* DNA mit den Primern FD3 und FD4 amplifiziert. Dem Primer FD3 wurden die mit Fettdruck hervorgehobenen Nukleotide für einen BamHl Ort angefügt. Durch Insertion eines C (Fett) wurde im Unterschied zur genomischen Sequenz ein BamHl Ort im Primer FD4 eingeführt.
Primer FD3: (SEQ ID NO: 5)
   5'-acggatccgagagacagagagacggagacaaaa-3'
Primer FD4: (SEQ ID NO: 6)
   5'-gcggatccaagcttcactgcttaaattc-3'

### Reaktionsansatz:

| | |
|---|---|
| 1µl | DNA |
| 37µl | H₂O |
| 5µl | 10x Puffer |
| 1µl | FD3 Primer 10µM |
| 1µl | FD4 Primer 10µM |
| 4µl | dNTP 2,5mM |
| 1µl | Pfu Turbo- DNA Polymerase (Stratagene) |

### PCR-Bedingungen:

| | |
|---|---|
| 1 | Zyklus mit 5 min. bei 95°C |
| 25 | Zyklen mit, 52°C für 1 min, 72°C für 1 min. und 95°C für 30 sec |
| 1 | Zyklus mit 72°C für 10 min., |

anschließend Kühlung auf 4°C bis zur Weiterverarbeitung.

### b) Konstruktion der FD:GUS Expressionskassetten

Das den FD Promoter enthaltende PCR-Produkt wurde mit dem Restriktionsenzym BamHI gespalten und in den Vektor pGUSINT37 (SunGene), ebenfalls BamHI gespalten, ligiert. Aus der ungerichteten Klonierung entstanden die beiden Plasmide pFD+GUS und pFD-GUS, in denen das Promoterfragment in jeweils entgegengesetzten Orientierungen vor dem GUS Gen platziert ist. Das Plasmid pFD+GUS enthält den Promoter in der Transkriptionsrichtung des putativen Ferredoxin-Gens, das Plasmid pFD-GUS in der Orientierung des annotierten O-Acetylserin-thiollyase Gens (OASTL, Cystein Synthase).

### Beispiel 4: Herstellung von Vektoren zur gleichzeitigen Analyse beider Transkriptionsrichtungen des FD Promoters

Zur Analyse beider Expressionsrichtungen wurde in zwei Konstrukten die Gene des Selektionsmarkers Nptll und des Reporters Glucuronidase unter die Kontrolle des bidirektionalen Promoters gestellt. Dazu wurden die Plasmide pFD+GUS und pFD-GUS mit EcoRI/Sall gespalten und in den Vektor pS5NptllCat (Derivat des pSUN Vektors; WO 02/00900) kloniert. Das resultierende Plasmide UH200 (SEQ ID NO: 3) enthält das GUS Gen unter der Kontrolle der in Richtung des Ferredoxingens wirkenden transkriptionellen Elemente und das Nptll Gen unter der Kontrolle des in Richtung OASTL Gen wirkenden transkriptionellen Elemente. Im Plasmid UH201 (SEQ ID NO: 4) befindet sich das GUS Gen unter der Kontrolle der OASTL gerichteten Faktoren und das Nptll Gen unter der Kontrolle der das Ferredoxin-Gen steuernden Elemente (siehe Fig.1). Beide Konstrukte wurden in den Agrobakterien-Stamm GV3101 [pMP90] transformiert und entsprechend den Protokollen in Tabak und Raps transformiert.

### Beispiel: 5 Ergebnisse der Analyse der Kanamycin-Resistenz der transgenen Tabakpflanzen

Die selektive Regeneration der Tabakpflänzchen erfolgte auf 100mg/l Kanamycin. 86% der Explantate der mit dem Konstrukt UH200 transformiert waren, entwickelten Sprossknospen. Von den geschnittenen Sprossen bewurzelten sich auf Kanamycin enthaltenden Medium 89%, die nach PCR Analysen alle transgen waren. 70% der Explantate aus dem Transformationsexperiment mit UH201 entwickelten Sprossknospen, von denen 90% sich bewurzelten. Auch hier ergab die PCR Analyse, dass die Pflänzchen das entsprechende Konstrukt enthalten und somit transgen sind. Dieses Beispiel zeigt, dass beide Promotororientierungen in gleicher Weise für die Expression von Selektionsmarkern während selektiver Regeneration von Tabak geeignet sind.

### Beispiel 6: Ergebnisse der Analyse der Kanamycin-Resistenz der transgenen Rapspflanzen

Die selektive Regeneration der Rapssprosse erfolgte auf 18 mg/l Kanamycin. Die Transformationseffizienz betrug für das Konstrukt UH200 11 % und für UH201 10%. Gleichzeitig wurde die Transformationseffizienz unter der Kontrolle des Promotors der Nopalin Synthase mit 8% ermittelt. Dieses Beispiel zeigte das die selektive Regeneration sowohl unter der Kontrolle des Promotors in der OASTL Richtung (UH200) als auch in der FD Richtung (UH201) mit dem üblicherweise verwendeten nosP vergleichbar ist.

### Beispiel 7: GUS-Analyse der Gewebespezifität des bidirektionalen Promoters in den transgenen Tabak und Rapspflanzen.

In den transgenen Tabak und Rapspflanzen haben beide Promotororientierungen die gleichen Gewebespezifitäten mit Ausnahme in Pollen gezeigt (Tabelle 1). Während in Raps keine Aktivitäten in den Pollen gefunden wurden, zeigte der Tabakpollen eine deutliche Blaufärbung und damit eine Promoteraktivität. Die GUS Expression reguliert von beider Orientierungen wurde vorwiegend in grünem Gewebe gefunden. In Wurzeln und Blütenblättem konnte keine Expression nachgewiesen werden. Bereits in sehr jungen Stadien der Samenentwicklung im Raps konnte eine GUS-Aktivität detektiert werden.

**Tabelle: 1: Übersicht über die Gewebespezifitäten in Tabak und Raps.**

| Gewebe | | A | | B | C | D | E | F | G | H | I | J | K | L |
|---|---|---|---|---|---|---|---|---|---|---|---|---|---|---|
| | | siL | soL | | | | | | | | | | | |
| Tabak | FD | ++ | ++ | - | + | + | ++ | ++ | + | + | ++ | - | + | ++ |
| | OASTL | + | + | - | + | + | + | + | + | + | ++ | - | + | ++ |
| Raps | FD | ++ | ++ | - | ++ | nd | + | + | + | + | + | - | + | - |

| | | | | | | | | | | | | | | |
|---|---|---|---|---|---|---|---|---|---|---|---|---|---|---|
| ++ hohe Aktivität + geringere Aktivität - keine Aktivität; nd: nicht bestimmt A Blätter (silL: "Sink" Blätter, soL: "SourceD-Blätter) B Wurzeln C Samen D Keimling E Stängel F Blütenstiele G Nodien H Knospe I Kelchblätter J Blütenblätter K Antheren L Pollen. | | | | | | | | | | | | | | |

Zur Verfolgung der Promotoraktivität während der selektiven Regeneration wurden junge Sprosse mit X-Gluc gefärbt. Transgene Sprosse zeigten eine starke Blaufärbung. Dieses Experiment zeigte wieder die gleiche Aktivität des bidirektionalen Promoters in beiden Orientierungen.

### Beispiel 8: Quantitative GUS-Analyse des bidirektionalen Promoters in den transgenen Tabakpflanzen

Zur quantitativen Analyse der Stärke des FD Promoters wurde parallel von transgenen Pflanzen beider Konstrukte Blatt und Samenmaterial untersucht. Der quantitative GUS Assay wurde entsprechend der Vorschrift von Jefferson mit MUG und 4-Methylumbelliferon als Standard durchgeführt. In den Samen der Pflanzen beider Orientierungen wurde eine ähnliche Menge an GUS Aktivität detektiert. Im Blattmaterial war die Expression in beiden Richtungen deutlich messbar, in der Intensität jedoch weniger uniform als im Samenmaterial.

### Beispiel 9: Quantitative GUS-Analyse des bidirektionalen Promoters in den transgenen Rapspflanzen

Raps wurde - wie oben beschrieben - ebenfalls mit den Konstrukten UH200 und UH201 transformiert. Eine quantitative GUS Analyse von Blattmaterial transgener Rapspflanzen zeigte, dass beide Promotorrichtungen eine gleiche Aktivität zeigten. In Fig. 2 sind die Werte der einzelnen Linien dargestellt. Die Höhe der Expression entspricht den anderen polarer pflanzlicher Promotoren.

## Patentansprüche

1. Transgene Expressionskassetten zur Expression von zwei Nukleinsäurensequenzen in einer pflanzlichen Zelle umfassend mindestens eine regulatorische Sequenz ausgewählt aus der Gruppe bestehend aus
a) dem Promotor gemäß SEQ ID NO: 1 oder 2,
b) funktionellen Äquivalenten des Promotor gemäß SEC ID NO: 1 oder 2, die eine Identität von mindestens 80% zu der Sequenz gemäß SEC ID NO: 1 oder 2 aufweisen und die im wesentlichen die gleiche Promotoraktivität wie der Promotor gemäß SEC ID NO: 1 oder 2 besitzen,
c) funktionellen Äquivalenten des Promotor gemäß SEQ ID NO: 1 oder 2, welche mindestens 25 aufeinanderfolgende Nukleotide der Sequenzen gemäß SEQ ID NO: 1 oder 2 umfassen und die im wesentlichen die gleiche Promotoraktivität wie der Promotor gemäß SEQ ID NO: 1 oder 2 besitzen, und
d) funktionell äquivalente Fragmente der Sequenzen a) oder b) oder c), die mindestens 25 aufeinanderfolgende Nukleotide besagter Sequenzen a) oder b) oder c) aufweisen und im wesentlichen die gleiche Promotoraktivität wie der Promotor gemäß SEQ ID NO: 1 oder 2 besitzen,
wobei besagte regulatorische Sequenz zwischen zwei Nukleinsäuresequenzen angeordnet ist und in Bezug auf besagte Nukleinsäuresequenzen heterolog ist - und mit besagten Nukleinsäuresequenzen funktionell so verknüpft ist, dass in mindestens einer pflanzlichen Zelle die Expression von zwei unterschiedlichen Ribonukleinsäuresequenzen bewirkt wird, wobei besagte Ribonukleinsäuresequenzen ausgewählt sind aus Ribonukleinsäuresequenzen kodierend für
i) Aminosäuresequenzen oder
ii) Ribonukleinsäuresequenzen, die eine Verminderung der Expression mindestens eines endogenen Gens besagter pflanzlichen Zelle bewirken.

2. Expressionskassette nach Anspruch 1, wobei die beiden transgen zu exprimierenden Nukleinsäuresequenzen unterschiedlich sind und für eine der folgenden Kombinationen kodieren
i) Selektionsmarker und Reporterprotein
ii) Zielprotein und Selektionsmarker oder Reporterprotein
ii) Zwei Zielproteine aus dem gleichen Stoffwechselweg
iii) Sense und antisense RNA
iv) Verschiedene Proteine zur Pathogenabwehr

3. Expressionskassette nach Anspruch 1 oder 2, wobei mindestens eine der transgen zu exprimierenden Nukleinsäuresequenzen ausgewählt ist aus Nukleinsäuren kodierend für Selektionsmarker, Reportergene, Cellulasen, Chitinasen, Glucanasen, Ribosom-inaktivierende Proteine, Lysozyme, Bacillus thuringiensis Endotoxinen, α-Amylaseinhibitoren, Proteaseinhibitoren, Lektinen, RNAasen, Ribozymen, Acetyl-CoA-Carboxylasen, Phytasen, 2S Albumin aus Bertholletia excelsa, "antifreeze"-Proteinen, Trehalosephosphatsynthasen, Trehalosephosphatphosphatasen, Trehalasen, DREB1A-Faktor, Farnesyltransferasen, Ferritin, Oxalatoxidasen, Calcium-abhängigen Proteinkinasen, Calcineurinen, Glutamatdehydrogenasen, N-hydroxylierende, multifunktionelle Cytochrom P-450, transkriptioneller Aktivator CBF1, Phytoendesaturasen, Polygalakturonasen, Flavonoid-3'-hydroxylasen, Dihydroflavanol-4-reduktasen, Chalconisomerasen, Chalconsynthasen, Flavanone-3-beta-hydroxylasen, Flavonsynthase II, Verzweigungsenzyms Q, "Starch Branching" Enzyme.

4. Transgene Expressionskassette nach einem der Ansprüche 1 bis 3, wobei mindestens eine der transgen zu exprimierenden Nukleinsäuresequenzen ausgewählt ist aus der Gruppe bestehend aus positiven Selektionsmarkern, negativen Selektionsmarkern und Faktoren die einen Wachstumsvorteil gewähren.

5. Transgene Expressionskassette nach Anspruch 2 oder 4, wobei der Selektionsmarker ausgewählt ist aus der Gruppe bestehend aus Proteinen, die eine Resistenz gegen Antibiotika, Metabolismus-Inhibitoren, Herbizide oder Biozide verleihen.

6. Transgene Expressionskassette nach einem der Ansprüche 2, 4 oder 5, wobei der Selektionsmarker ausgewählt ist aus der Gruppe bestehend aus Proteinen, die eine Resistenz verleihen gegen Phosphinothricin, Glyphosat, Bromoxynil, Dalapon, 2-Desoxyglucose-6-phosphat, Tetracyclin, Ampicillin, Kanamycin, G 418, Neomycin, Paromomycin, Bleomycin, Zeocin, Hygromycin, Chloramphenicol, Sulfonylharnstoff-Herbizide, Imidazolinon-Herbizide.

7. Transgene Expressionskassette nach einem der Ansprüche 2 oder 4 bis 6, wobei der Selektionsmarker ausgewählt ist aus der Gruppe bestehend aus Phosphinothricinacetyltransferasen, 5-Enolpyruvylshikimat-3-phosphatsynthasen, Glyphosatoxidoreduktasen, Dehalogenase, Nitrilasen, Neomycinphosphotransferasen, DOG^{R}1-Genen, Acetolactatsynthasen, Hygromycinphosphotransferasen, Chloramphenicolacetyltransferasen, Streptomycinadenylyltransferasen, β-Lactamasen, tetA Genen, tetR Genen, Isopentenyltransferasen, Thymidinkinasen, Diphtheriatoxin A, Cytosindeaminase (codA), Cytochrom P450, Haloalkandehalogenasen, iaaH Gene, tms2 Gene, β-Glucuronidasen, Mannose-6-phosphat-Isomerasen, UDP-Galaktose-4-Epimerasen.

8. Transgener Expressionsvektor enthaltend eine Expressionskassette gemäß einem der Ansprüche 1 bis 7.

9. Transgener pflanzliches Organismus transformiert mit einer transgenen Expressionskassette gemäß einem der Ansprüche 1 bis 7 oder einem transgenen Expressionsvektor gemäß Anspruch 8.

10. Transgener pflanzliches Organismus nach Anspruch 9, ausgewählt aus der Gruppe bestehend aus Arabidopsis, Tomate, Tabak, Kartoffeln, Mais, Raps, Weizen, Gerste, Sonnenblumen, Hirse, Rübe, Roggen, Hafer, Zuckerrübe, Bohnengewächse und Soja.

11. Zelle, Zellkulturen, Teile oder transgenes Vermehrungsgut abgeleitet von einem transgenen pflanzlichen Organismus nach Anspruch 9 oder 10.

12. Verfahren zur transgenen Expression von zwei Ribonukleinsäuresequenzen in pflanzlichen Zellen, wobei eine Expressionskassetten umfassend mindestens eine regulatorische Sequenz ausgewählt aus der Gruppe bestehend aus
a) dem Promotor gemäß SEQ ID NO: 1 oder 2,
b) funktionellen Äquivalenten des Promotor gemäß SEC ID NO: 1 oder 2, die eine Identität von mindestens 80% zu der Sequenz gemäß SEQ ID NO: 1 oder 2 aufweisen und die im wesentlichen die gleiche Promotoraktivität wie der Promotor gemäß SEC ID NO: 1 oder 2 besitzen,
c) funktionellen Äquivalenten des Promotor gemäß SEQ ID NO: 1 oder 2, welche mindestens 25 aufeinanderfolgende Nukleotide der Sequenzen gemäß SEQ ID NO: 1 oder 2 umfassen und die im wesentlichen die gleiche Promotoraktivität wie der Promotor gemäß SEQ ID NO: 1 oder 2 besitzen, und
d) funktionell äquivalente Fragmente der Sequenzen a) oder b) oder c), die mindestens 25 aufeinanderfolgende Nukleotide besagter Sequenzen a) oder b) oder c) aufweisen und im wesentlichen die gleiche Promotoraktivität wie der Promotor gemäß SEQ ID NO: 1 oder 2 besitzen,
in mindestens eine pflanzliche Zelle eingebracht wird,
wobei besagte regulatorische Sequenz zwischen zwei Nukleinsäuresequenzen angeordnet ist und in Bezug auf besagte Nukleinsäuresequenz heterolog ist und mit besagten Nukleinsäuresequenzen funktionell so verknüpft ist, dass in mindestens besagter pflanzlichen Zelle die Expression besagter zwei unterschiedlichen Ribonukleinsäuresequenzen bewirkt wird, wobei besagte Ribonukleinsäuresequenzen ausgewählt sind aus Ribonukleinsäuresequenzen kodierend für
i) Aminosäuresequenzen oder
ii) Ribonukleinsäuresequenzen, die eine Verminderung der Expression mindestens eines endogenen Gens besagter pflanzlichen Zelle bewirken.

13. Verfahren nach Anspruch 12, wobei die beiden transgen zu exprimierenden Nukleinsäuresequenzen unterschiedlich sind und für eine der folgenden Kombinationen kodieren
i) Selektionsmarker und Reporterprotein
ii) Zielprotein und Selektionsmarker oder Reporterprotein
ii) Zwei Zielproteine aus dem gleichen Stoffwechselweg
iii) Sense und antisense RNA
iv) Verschiedene Proteine zur Pathogenabwehr

14. Verfahren nach Anspruch 12 oder 13, wobei mindestens eine der transgen zu exprimierende Nukleinsäuresequenz ausgewählt ist aus Nukleinsäuren wie definiert in einem der Ansprüche 3 bis 7.

15. Verwendung eines transgenen pflanzlichen Organismus nach Anspruch 9 oder 10 oder von diesem abgeleitete Zellkulturen, Teile oder transgenes Vermehrungsgut nach Anspruch 11 zur Herstellung von Nahrungs-, Futtermitteln, Saatgut, Pharmazeutika oder Feinchemikalien.

16. Verwendung nach Anspruch 15, wobei die Feinchemikalien Antikörper, Enzyme, pharmazeutisch aktive Proteine, Vitamine, Aminosäuren, Zucker, gesättigte oder ungesättigte Fettsäuren, natürliche oder synthetische Geschmacks-, Aroma- oder Farbstoffe sind.

17. Verfahren zur Herstellung von Pharmazeutika oder Feinchemikalien in transgenen pflanzlichen Organismen nach Anspruch 9 oder 10 oder von diesen abgeleiteten Zellkulturen, Teilen oder transgenes Vermehrungsgut nach Anspruch 11, **dadurch gekennzeichnet, dass** der transgene pflanzliche Organismus gezüchtet und das gewünschte Pharmazeutikum oder die gewünschte Feinchemikalie isoliert wird.

## Claims

1. A transgenic expression cassette for expressing two nucleic acid sequences in a plant cell comprising at least one regulatory sequence selected from the group consisting of
a) the promoter shown in SEQ ID NO: 1 or 2,
b) functional equivalents of the promoter shown in SEQ ID NO: 1 or 2 which have an identity of at least 80% to the sequence shown in SEQ ID NO: 1 or 2 and which have substantially the same promoter activity as the promoter shown in SEQ ID NO: 1 or 2,
c) functional equivalents of the promoter shown in SEQ ID NO: 1 or 2 which comprise at least 25 consecutive nucleotides of the sequences shown in SEQ ID NO: 1 or 2 and which have substantially the same promoter activity as the promoter shown in SEQ ID NO: 1 or 2, and
d) functionally equivalent fragments of sequences a) or b) or c), which have at least 25 consecutive nucleotides of said sequences a) or b) or c) and have substantially the same promoter activity as the promoter shown in SEQ ID NO: 1 or 2,
where said regulatory sequence is disposed between two nucleic acid sequences and is heterologous in relation to said nucleic acid sequences and is functionally linked to said nucleic acid sequences in such a way that the expression of two different ribonucleic acid sequences is brought about in at least one plant cell, where said ribonucleic acid sequences are selected from ribonucleic acid sequences coding for
i) amino acid sequences or
ii) ribonucleic acid sequences which bring about a reduction in the expression of at least one endogenous gene of said plant cell.

2. The expression cassette according to claim 1, where the two nucleic acid sequences to be expressed transgenically are different and code for one of the following combinations
i) selection marker and reporter protein
ii) target protein and selection marker or reporter protein
ii) two target proteins from the same metabolic pathway
iii) sense and antisense RNA
iv) various proteins for defense against pathogens

3. The expression cassette according to claim 1 or 2, where at least one of the nucleic acid sequences to be expressed transgenically is selected from nucleic acids coding for selection markers, reporter genes, cellulases, chitinases, glucanases, ribosome-inactivating proteins, lysozymes, Bacillus thuringiensis endotoxins, □^{~}amylase inhibitors, protease inhibitors, lectins, RNAases, ribozymes, acetyl-CoA carboxylases, phytases, 2S albumin from Bertholletia excelsa, antifreeze proteins, trehalose-phosphate synthases, trehalose-phosphate phosphatases, trehalases, DREB1A factor, farnesyltransferases, ferritin, oxalate oxidases, calcium-dependent protein kinases, calcineurins, glutamate dehydrogenases, N-hydroxylating multifunctional cytochrome P-450, transcriptional activator CBF1, phytoene desaturases, polygalacturonases, flavonoid 3'-hydroxylases, dihydroflavanol 4-reducases, chalcone isomerases, chalcone synthases, flavanone 3-beta-hydroxylases, flavone synthase II, branching enzyme Q, starch branching enzymes.

4. The transgenic expression cassette according to any of claims 1 to 3, where at least one of the nucleic acid sequences to be expressed transgenically is selected from the group consisting of positive selection markers, negative selection markers and factors which provide a growth advantage.

5. The transgenic expression cassette according to claim 2 or 4, where the selection marker is selected from the group consisting of proteins which confer a resistance to antibiotics, metabolism inhibitors, herbicides or biocides.

6. The transgenic expression cassette according to any of claims 2, 4 or 5, where the selection marker is selected from the group consisting of proteins which confer a resistance to phosphinothricin, glyphosate, bromoxynil, dalapon, 2-deoxyglucose 6-phosphate, tetracycline, ampicillin, kanamycin, G 418, neomycin, paromomycin, bleomycin, zeocin, hygromycin, chloramphenicol, sulfonylurea herbicides, imidazolinone herbicides.

7. The transgenic expression cassette according to any of claims 2 or 4 to 6, where the selection marker is selected from the group consisting of phosphinothricin acetyltransferases, 5-enolpyruvylshikimate-3-phosphate synthases, glyphosate oxidoreductases, dehalogenase, nitrilases, neomycin phosphotransferases, DOG^{R}1 genes, acetolactate synthases, hygromycin phosphotransferases, chloramphenicol acetyltransferases, streptomycin adenylyltransferases, β-lactamases, tetA genes, tetR genes, isopentenyltransferases, thymidine kinases, diphtheria toxin A, cytosine deaminase (codA), cytochrome P450, haloalkane dehalogenases, iaaH genes, tms2 genes, β-glucuronidases, mannose-6-phosphate isomerases, UDP-galactose 4-epimerases.

8. A transgenic expression vector comprising an expression cassette according to any of claims 1 to 7.

9. A transgenic plant organism transformed with a transgenic expression cassette according to any of claims 1 to 7 or with a transgenic expression vector according to claim 8.

10. The transgenic plant organism according to claim 9, selected from the group consisting of arabidopsis, tomato, tobacco, potatoes, corn, oilseed rape, wheat, barley, sunflowers, millet, beet, rye, oats, sugarbeet, beans and soybean.

11. A cell, cell culture, part or transgenic propagation material derived from a transgenic plant organism according to claim 9 or 10.

12. A process for transgenic expression of two ribonucleic acid sequences in plant cells, where an expression cassette comprising at least one regulatory sequence selected from one group consisting of
a) the promoter shown in SEQ ID NO: 1 or 2,
b) functional equivalents of the promoter shown in SEQ ID NO: 1 or 2 which have an identity of at least 80% to the sequence shown in SEQ ID NO: 1 or 2 and which have substantially the same promoter activity as the promoter shown in SEQ ID NO: 1 or 2,
c) functional equivalents of the promoter shown in SEQ ID NO: 1 or 2 which comprise at least 25 consecutive nucleotides of the sequences shown in SEQ ID NO: 1 or 2 and which have substantially the same promoter activity as the promoter shown in SEQ ID NO: 1 or 2, and
d) functionally equivalent fragments of sequences a) or b) or c), which have at least 25 consecutive nucleotides of said sequences a) or b) or c) and have substantially the same promoter activity as the promoter shown in SEQ ID NO: 1 or 2,
is introduced into at least one plant cell,
where said regulatory sequence is disposed between two nucleic acid sequences and is heterologous in relation to said nucleic acid sequence and is functionally linked to said nucleic acid sequences in such a way that the expression of said two different ribonucleic acid sequences is brought about in at least said plant cell, where said ribonucleic acid sequences are selected from ribonucleic acid sequences coding for
i) amino acid sequences or
ii) ribonucleic acid sequences which bring about a reduction in the expression of at least one endogenous gene of said plant cell.

13. The process according to claim 12, where the two nucleic acid sequences to be expressed transgenically are different and code for one of the following combinations
i) selection marker and reporter protein
ii) target protein and selection marker or reporter protein
ii) two target proteins from the same metabolic pathway
iii) sense and antisense RNA
iv) various proteins for defense against pathogens

14. The process according to claim 12 or 13, where at least one of the nucleic acid sequences to be expressed transgenically is selected from nucleic acids as defined in any of claims 3 to 7.

15. The use of a transgenic plant organism according to claim 9 or 10 or of cell cultures, parts or transgenic propagation material derived therefrom according to claim 11 for producing human or animal foods, seeds, pharmaceuticals or fine chemicals.

16. The use according to claim 15, where the fine chemicals are antibodies, enzymes, pharmaceutically active proteins, vitamins, amino acids, sugars, saturated or unsaturated fatty acids, natural or synthetic flavorings, aromatizing substances or colorants.

17. A process for producing pharmaceuticals or fine chemicals in transgenic plant organisms according to claim 9 or 10 or cell cultures, parts or transgenic propagation material derived therefrom according to claim 11, which comprises culturing the transgenic plant organism and isolating the desired pharmaceutical or the desired fine chemical.

## Revendications

1. Cassettes d'expression transgénique pour l'expression de deux séquences d'acide nucléique dans une cellule végétale, comprenant au moins une séquence de régulation choisie dans le groupe constitué :
a) du promoteur représenté par la SEQ ID n° 1 ou 2,
b) d'équivalents fonctionnels du promoteur représenté par la SEQ ID n° 1 ou 2, qui présentent au moins 80 % d'identité par rapport à la séquence représentée par la SEQ ID n° 1 ou 2 et qui présentent sensiblement la même activité de promoteur que celle du promoteur représenté par la SEQ ID n° 1 ou 2,
c) d'équivalents fonctionnels du promoteur représenté par la SEQ ID n° 1 ou 2, qui comprennent au moins 25 nucléotides successifs de la séquence représentée par la SEQ ID n° 1 ou 2 et qui présentent sensiblement la même activité de promoteur que celle du promoteur représenté par la SEQ ID n° 1 ou 2, et
d) de fragments équivalents sur le plan fonctionnel des séquences définies au point a) ou b) ou c), qui présentent au moins 25 nucléotides successifs desdites séquences définies au point a) ou b) ou c) et qui présentent sensiblement la même activité de promoteur que celle du promoteur représenté par la SEQ ID n° 1 ou 2,
ladite séquence de régulation étant placée entre deux séquences d'acide nucléique, étant hétérologue vis-à-vis desdites séquences d'acide nucléique et étant reliée de manière fonctionnelle auxdites séquences d'acide nucléique de manière à provoquer l'expression de deux séquences d'acide ribonucléique différentes dans au moins une cellule végétale, et lesdites séquences d'acide ribonucléique étant choisies parmi des séquences d'acide ribonucléique codant pour :
i) des séquences d'acides aminés ou
ii) des séquences d'acide ribonucléique qui provoquent une diminution de l'expression d'au moins un gène endogène de ladite cellule végétale.

2. Cassette d'expression selon la revendication 1, dans laquelle les deux séquences d'acide nucléique, que l'on souhaite exprimer par voie transgénique, sont différentes et codent pour l'une des combinaisons suivantes :
i) un marqueur de sélection et une protéine rapporteur,
ii) une protéine cible et un marqueur de sélection ou une protéine rapporteur,
ii) deux protéines cibles provenant de la même voie métabolique,
iii) un ARN sens et un ARN antisens,
iv) diverses protéines de défense contre les agents pathogènes.

3. Cassette d'expression selon la revendication 1 ou 2, dans laquelle au moins une des séquences d'acide nucléique, que l'on souhaite exprimer par voie transgénique, est choisie à partir d'acides nucléiques codant pour des marqueurs de sélection, des gènes rapporteurs, des cellulases, des chitinases, des glucanases, des protéines d'inactivation du ribosome, des lysozymes, des endotoxines de l'espèce *Bacillus thuringiensis,* des inhibiteurs de la α-amylase, des inhibiteurs de la protéase, des lectines, des ARNases, des ribozymes, des acétyl-CoA carboxylases, des phytases, de l'albumine 2S issue de l'espèce *Bertholletia excelsa,* des protéines "antigel", des tréhalose-phosphate synthases, des tréhalose-phosphate phosphatases, des tréhalases, du facteur DREB1A, des farnésyle transférases, de la ferritine, des oxalate oxydases, des protéinekinases dépendantes du calcium, des calcineurines, des glutamate déshydrogénases, du cytochrome P-450 multifonctionnel à effet d'hydroxylation d'azote, de l'activateur transcriptionnel CBF1, des phytoène désaturases, des polygalacturonases, des flavonoïde-3'-hydroxylases, des dihydroflavanol-4-réductases, des chalcone isomérases, des chalcone synthases, des flavanone-3-bêta-hydroxylases, des flavone synthases II, des enzymes de ramification Q et des enzymes de "ramification d'amidon".

4. Cassette d'expression transgénique selon l'une quelconque des revendications 1 à 3, dans laquelle au moins une des séquences d'acide nucléique, que l'on souhaite exprimer par voie transgénique, est choisie dans le groupe constitué des marqueurs de sélection positifs, des marqueurs de sélection négatifs et des facteurs qui garantissent un avantage en matière de croissance.

5. Cassette d'expression transgénique selon la revendication 2 ou 4, dans laquelle le marqueur de sélection est choisi dans le groupe constitué des protéines qui confèrent une résistance aux antibiotiques, aux inhibiteurs de métabolisme, aux herbicides ou aux biocides.

6. Cassette d'expression transgénique selon l'une quelconque des revendications 2, 4 ou 5, dans laquelle le marqueur de sélection est choisi dans le groupe constitué des protéines qui confèrent une résistance à la phosphinothricine, au glyphosate, au bromoxynile, au dalapon, au 2-désoxyglucose-6-phosphate, à la tétracycline, à l'ampicilline, à la kanamycine, à la G418, à la néomycine, à la paromomycine, à la bléomycine, à la zéocine, à l'hygromycine, au chloramphénicol, aux herbicides à base de sulfonylurée et aux herbicides à base d'imidazolinone.

7. Cassette d'expression transgénique selon l'une quelconque des revendications 2 ou 4 à 6, dans laquelle le marqueur de sélection est choisi dans le groupe constitué des phosphinothricine acétyltransférases, des 5-énolpyruvylshikimate-3-phosphate synthases, des glyphosate oxydoréductases, des déshalogénases, des nitrilases, des néomycine phosphotransférases, des gènes DOG^{R}1, des acétolactate synthases, des hygromycine phosphotransférases, des chloramphénicol acétyltransférases, des streptomycine adénylyltransférases, des β-lactamases, des gènes tetA, des gènes tetR, des isopentényltransférases, des thymidine kinases, de la toxine diphtérique A, de la cytosine désaminase (codA), du cytochrome P450, des halogénoalcane déshalogénases, des gènes iaaH, des gènes tms2, des β-glucuronidases, des mannose-6-phosphate-isomérases, des UDP-galactose-4-épimérases.

8. Vecteur d'expression transgénique contenant une cassette d'expression selon l'une quelconque des revendications 1 à 7.

9. Organisme végétal transgénique transformé par une cassette d'expression transgénique selon l'une quelconque des revendications 1 à 7, ou par un vecteur d'expression transgénique selon la revendication 8.

10. Organisme végétal transgénique selon la revendication 9, choisi dans le groupe constitué des variétés arabidopsis, tomate, tabac, pomme de terre, maïs, colza, blé, orge, tournesol, millet, betterave, seigle, avoine, betterave sucrière, plants de haricots et soja.

11. Cellule, cultures cellulaires, parties ou produits d'amplification transgéniques dérivés d'un organisme végétal transgénique selon la revendication 9 ou 10.

12. Procédé d'expression transgénique de deux séquences d'acide ribonucléique dans des cellules végétales, dans lequel on introduit une cassette d'expression comprenant au moins une séquence de régulation choisie dans le groupe constitué :
a) du promoteur représenté par la SEQ ID n° 1 ou 2,
b) d'équivalents fonctionnels du promoteur représenté par la SEQ ID n° 1 ou 2, qui présentent au moins 80 % d'identité par rapport à la séquence représentée par la SEQ ID n° 1 ou 2 et qui possèdent sensiblement la même activité de promoteur que celle du promoteur représenté par la SEQ ID n° 1 ou 2,
c) d'équivalents fonctionnels du promoteur représenté par la SEQ ID n° 1 ou 2, qui comprennent au moins 25 nucléotides successifs de la séquence représentée par la SEQ ID n° 1 ou 2 et qui possèdent sensiblement la même activité de promoteur que celle du promoteur représenté par la SEQ ID n° 1 ou 2, et
d) de fragments équivalents sur le plan fonctionnel des séquences définies au point a) ou b) ou c), qui présentent au moins 25 nucléotides successifs desdites séquences définies au point a) ou b) ou c) et qui présentent sensiblement la même activité de promoteur que celle du promoteur représenté par la SEQ ID n° 1 ou 2,
dans au moins une cellule végétale,
ladite séquence de régulation étant disposée entre deux séquences d'acide nucléique, étant hétérologue vis-à-vis de ladite séquence d'acide nucléique et étant reliée de manière fonctionnelle auxdites séquences d'acide nucléique de manière à provoquer l'expression desdites deux séquences d'acide ribonucléique différentes dans au moins ladite cellule végétale, lesdites séquences d'acide ribonucléique étant choisies parmi des séquences d'acide ribonucléique codant pour :
i) des séquences d'acides aminés ou
ii) des séquences d'acide ribonucléique qui provoquent une diminution de l'expression d'au moins un gène endogène de ladite cellule végétale.

13. Procédé selon la revendication 12, dans lequel les deux séquences d'acide nucléique, que l'on souhaite exprimer par voie transgénique, sont différentes et codent pour l'une des combinaisons suivantes :
i) un marqueur de sélection et une protéine rapporteur,
ii) une protéine cible et un marqueur de sélection ou une protéine rapporteur,
iii) deux protéines cibles provenant de la même voie métabolique,
iv) un ARN sens et un ARN antisens,
v) diverses protéines de défense contre les agents pathogènes.

14. Procédé selon la revendication 12 ou 13, dans lequel on choisit au moins une des séquences d'acide nucléique, que l'on souhaite exprimer par voie transgénique, parmi les acides nucléiques tels que définis selon l'une quelconque des revendications 3 à 7.

15. Utilisation d'un organisme végétal transgénique selon la revendication 9 ou 10, ou de cultures cellulaires, parties ou produits d'amplification transgéniques dérivés de celui-ci selon la revendication 11, pour fabriquer des produits alimentaires, des aliments pour animaux, des semences, des produits pharmaceutiques ou des produits chimiques fins.

16. Utilisation selon la revendication 15, dans laquelle les produits chimiques fins sont des anticorps, des enzymes, des protéines actives sur le plan pharmaceutique, des vitamines, des acides aminés, des sucres, des acides gras saturés ou insaturés, des substances sapides, des arômes ou des colorants naturels ou synthétiques.

17. Procédé de fabrication de produits pharmaceutiques ou de produits chimiques fins dans des organismes végétaux transgéniques selon la revendication 9 ou 10, ou de cultures cellulaires, parties ou produits d'amplification transgéniques dérivés de ceux-ci selon la revendication 11, **caractérisé en ce que** l'on cultive l'organisme végétal transgénique et **en ce que** l'on isole le produit pharmaceutique ou le produit chimique fin souhaité.


### SEQUENCE LISTING

<110> SunGene GmbH&Co.KGaA
   <120> Expressionskassetten zur bidirektionale transgenen Expression von Nukleinsäuren in Pflanzen
   <130> AE 20030535
   <160> 6
   <170> PatentIn version 3.1
<210> 1
   <211> 429
   <212> DNA
   <213> Arabidopsis thaliana
<220>
   <221> promoter
   <222> (1)..(429)
   <223>
<400> 1
   gtatggaata aaatcttcga atgatgagat atatgatctc tttggtgtca gtcacatggc     60
   acacgctatc aatttagaaa aacgcggtgg ttggtcacca gaattactac ttctcggtct    120
   gatttggtca tatccgtatt aagtccggtt aatattttcc ataactgggg tttgaacatt    180
   cggtttcttt ttttcagtta gtccgatttg gagttttgag tatggaaaaa taatactgaa    240
   tttatttgtt caaactgttt tggaaaaaat atttccctta attacgaata taattaaaat    300
   tttaaaattc attttattag atcttggtta attcggttta atgcattaat gaatttcggt    360
   ttaagtcggt tttcggtttt tatgtcccac cactatctac aaccgatgat caaccttatc    420
   tccgtattc                                                            429
<210> 2
   <211> 836
   <212> DNA
   <213> Arabidopsis thaliana
<220>
   <221> promoter
   <222> (344)..(772)
   <223>
<220>
   <221> Intron
   <222> (14)..(281)
   <223> 1st intron of OASTL gene
<220>
   <221> 5'UTR
   <222> (773)..(836)
   <223> 5'UTR of FD gene
<220>
   <221> 5'UTR
   <222> (1)..(343)
   <223> 5'-UTR of OASTL gene comprising intron
<400> 2
   gatccaagct tcactgctta aattcacaaa aagagaaaag taagaccaaa ggaataaatc    60
   atcctcaaac caaaaacaca tcatacaaaa tcatcaaaca taaatctcca gatgtatgag   120
   caccaatcca gttatacaac actcttaaca ccaaatcaac agatttaaca gcgaaataag   180
   cttaagccca tacaattatc cgatccaaac aaatataatc gaaaccggca gaggaataag   240
   caagtgaatc aaaaagtatg ggacgaggaa gaagatgata cctgaatgag aaagtcaata   300
   accttgaccc gaatcgtttt gaagaaaatg gagaaaatcg gttgtatgga ataaaatctt   360
   cgaatgatga gatatatgat ctctttggtg tcagtcacat ggcacacgct atcaatttag   420
   aaaaacgcgg tggttggtca ccagaattac tacttctcgg tctgatttgg tcatatccgt   480
   attaagtccg gttaatattt tccataactg gggtttgaac attcggtttc tttttttcag   540
   ttagtccgat ttggagtttt gagtatggaa aaataatact gaatttattt gttcaaactg   600
   ttttggaaaa aatatttccc ttaattacga atataattaa aattttaaaa ttcattttat   660
   tagatcttgg ttaattcggt ttaatgcatt aatgaatttc ggtttaagtc ggttttcggt   720
   ttttatgtcc caccactatc tacaaccgat gatcaacctt atctccgtat tcaccacaaa   780
   cagtcatcac tctcacttga cacaaaaact cttttgtctc cgtctctctg tctctc        836
<210> 3
   <211> 11533
   <212> DNA
   <213> Artificial Sequence
<220>
   <223> Expression vector UH200
<400> 3
   ttccatggac atacaaatgg acgaacggat aaaccttttc acgccctttt aaatatccga    60
   ttattctaat aaacgctctt ttctcttagg tttacccgcc aatatatcct gtcaaacact   120
   gatagtttaa actgaaggcg ggaaacgaca atcagatcta gtaggaaaca gctatgacca   180
   tgattacgcc aagcttgcat gccgatcccc cccactccgc cctacactcg tatatatatg   240
   cctaaacctg ccccgttcct catatgtgat attattattt cattattagg tataagatag   300
   taaacgataa ggaaagacaa tttattgaga aagccatgct aaaatataga tagatatacc   360
   ttagcaggtg tttattttac aacataacat aacatagtag ctagccagca ggcaggctaa   420
   aacatagtat agtctatctg cagggggtac ggtcgactct agactagtgg atccgtcgaa   480
   gctagcttgg gtcccgctca gaagaactcg tcaagaaggc gatagaaggc gatgcgctgc   540
   gaatcgggag cggcgatacc gtaaagcacg aggaagcggt cagcccattc gccgccaagc   600
   tcttcagcaa tatcacgggt agccaacgct atgtcctgat agcggtccgc cacacccagc   660
   cggccacagt cgatgaatcc agaaaagcgg ccattttcca ccatgatatt cggcaagcag 720
   gcatcgccat gggtcacgac gagatcctcg ccgtcgggca tgcgcgcctt gagcctggcg 780
   aacagttcgg ctggcgcgag cccctgatgc tcttcgtcca gatcatcctg atcgacaaga 840
   ccggcttcca tccgagtacg tgctcgctcg atgcgatgtt tcgcttggtg gtcgaatggg 900
   caggtagccg gatcaagcgt atgcagccgc cgcattgcat cagccatgat ggatactttc 960
   tcggcaggag caaggtgaga tgacaggaga tcctgccccg gcacttcgcc caatagcagc 1020
   cagtcccttc ccgcttcagt gacaacgtcg agcacagctg cgcaaggaac gcccgtcgtg 1080
   gccagccacg atagccgcgc tgcctcgtcc tgcagttcat tcagggcacc ggacaggtcg 1140
   gtcttgacaa aaagaaccgg gcgcccctgc gctgacagcc ggaacacggc ggcatcagag 1200
   cagccgattg tctgttgtgc ccagtcatag ccgaatagcc tctccaccca agcggccgga 1260
   gaacctgcgt gcaatccatc ttgttcaatc caagctccca tgggccctcg actagagtcg 1320
   agatccgata tcgcccgggc tcgactctag aggatccaag cttcactgct taaattcaca 1380
   aaaagagaaa agtaagacca aaggaataaa tcatcctcaa accaaaaaca catcatacaa 1440
   aatcatcaaa cataaatctc cagatgtatg agcaccaatc cagttataca acactcttaa 1500
   caccaaatca acagatttaa cagcgaaata agcttaagcc catacaatta tccgatccaa 1560
   acaaatataa tcgaaaccgg cagaggaata agcaagtgaa tcaaaaagta tgggacgagg 1620
   aagaagatga tacctgaatg agaaagtcaa taaccttgac ccgaatcgtt ttgaagaaaa 1680
   tggagaaaat cggttgtatg gaataaaatc ttcgaatgat gagatatatg atctctttgg 1740
   tgtcagtcac atggcacacg ctatcaattt agaaaaacgc ggtggttggt caccagaatt 1800
   actacttctc ggtctgattt ggtcatatcc gtattaagtc cggttaatat tttccataac 1860
   tggggtttga acattcggtt tctttttttc agttagtccg atttggagtt ttgagtatgg 1920
   aaaaataata ctgaatttat ttgttcaaac tgttttggaa aaaatatttc ccttaattac 1980
   gaatataatt aaaattttaa aattcatttt attagatctt ggttaattcg gtttaatgca 2040
   ttaatgaatt tcggtttaag tcggttttcg gtttttatgt cccaccacta tctacaaccg 2100
   atgatcaacc ttatctccgt attcaccaca aacagtcatc actctcactt gacacaaaaa 2160
   ctcttttgtc tccgtctctc tgtctctcgg atccccgggt aggtcagtcc cttatgttac 2220
   gtcctgtaga aaccccaacc cgtgaaatca aaaaactcga cggcctgtgg gcattcagtc 2280
   tggatcgcga aaactgtgga attggtcagc gttggtggga aagcgcgtta caagaaagcc 2340
   gggcaattgc tgtgccagga gtttttaacg atcaagttcg ccgatgccag atattcgtaa 2400
   ttatgccggc aacgtcttgg tatcagcgcc gaagtcttta ttccgaaagg ttgggcaggc 2460
   cagcgtatcg tgctgcgttt cgatgcggtc actcattacg gcaaagtgtg ggtcaataat 2520
   caggaagtga tggagcatca gggcggctat acgccatttg aagccgatgt cacgccgtat 2580
   gttattgccg ggaaaagtgt acgtaagttt ctgcttctac ctttgatata tatataataa 2640
   ttatcattaa ttagtagtaa tataatattt caaatatttt tttcaaaata aaagaatgta 2700
   gtatatagca attgcttttc tgtagtttat aagtgtgtat attttaattt ataacttttc 2760
   taatatatga ccaaaatttg ttgatgtgca ggtatcaccg tttgtgtgaa caacgaactg 2820
   aactggcaga ctatcccgcc gggaatggtg attaccgacg aaaacggcaa gaaaaagcag 2880
   tcttacttcc atgatttctt taactatgcc ggaatccatc gcagcgtaat gctctacacc 2940
   acgccgaaca cctgggtgga cgatatcacc gtggtgacgc atgtcgcgca agactgtaac 3000
   cacgcgtctg ttgactggca ggtggtggcc aatggtgatg tcagcgttga actgcgtgat 3060
   gcggatcaac aggtggttgc aactggacaa ggcactagcg ggactttgca agtggtgaat 3120
   ccgcacctct ggcaaccggg tgaaggttat ctctatgaac tgtgcgtcac agccaaaagc 3180
   cagacagagt gtgatatcta cccgcttcgc gtcggcatcc ggtcagtggc agtgaagggc 3240
   gaacagttcc tgattaacca caaaccgttc tactttactg gctttggtcg tcatgaagat 3300
   gcggacttac gtggcaaagg attcgataac gtgctgatgg tgcacgacca cgcattaatg 3360
   gactggattg gggccaactc ctaccgtacc tcgcattacc cttacgctga agagatgctc 3420
   gactgggcag atgaacatgg catcgtggtg attgatgaaa ctgctgctgt cggctttaac 3480
   ctctctttag gcattggttt cgaagcgggc aacaagccga aagaactgta cagcgaagag 3540
   gcagtcaacg gggaaactca gcaagcgcac ttacaggcga ttaaagagct gatagcgcgt 3600
   gacaaaaacc acccaagcgt ggtgatgtgg agtattgcca acgaaccgga tacccgtccg 3660
   caagtgcacg ggaatatttc gccactggcg gaagcaacgc gtaaactcga cccgacgcgt 3720
   ccgatcacct gcgtcaatgt aatgttctgc gacgctcaca ccgataccat cagcgatctc 3780
   tttgatgtgc tgtgcctgaa ccgttattac ggatggtatg tccaaagcgg cgatttggaa 3840
   acggcagaga aggtactgga aaaagaactt ctggcctggc aggagaaact gcatcagccg 3900
   attatcatca ccgaatacgg cgtggatacg ttagccgggc tgcactcaat gtacaccgac 3960
   atgtggagtg aagagtatca gtgtgcatgg ctggatatgt atcaccgcgt ctttgatcgc 4020
   gtcagcgccg tcgtcggtga acaggtatgg aatttcgccg attttgcgac ctcgcaaggc 4080
   atattgcgcg ttggcggtaa caagaaaggg atcttcactc gcgaccgcaa accgaagtcg 4140
   gcggcttttc tgctgcaaaa acgctggact ggcatgaact tcggtgaaaa accgcagcag 4200
   ggaggcaaac aatgaatcaa caactctcct ggcgcaccat cgtcggctac agcctcggga 4260
   attgctaccg agctcggtac ccggcgcaaa aatcaccagt ctctctctac aaatctatct 4320
   ctctctattt ttctccagaa taatgtgtga gtagttccca gataagggaa ttagggttct 4380
   tatagggttt cgctcatgtg ttgagcatat aagaaaccct tagtatgtat ttgtatttgt 4440
   aaaatacttc tatcaataaa atttctaatt cctaaaacca aaatccagtg accgggtacc 4500
   gagctcgaat tcactggccg tcgttttaca acgactcagc agcttgacag gaggcccgat 4560
   ctagtaacat agatgacacc gcgcgcgata atttatccta gtttgcgcgc tatattttgt 4620
   tttctatcgc gtattaaatg tataattgcg ggactctaat cataaaaacc catctcataa 4680
   ataacgtcat gcattacatg ttaattatta catgcttaac gtaattcaac agaaattata 4740
   tgataatcat cgcaagaccg gcaacaggat tcaatcttaa gaaactttat tgccaaatgt 4800
   ttgaacgatc ggggatcatc cgggtctgtg gcgggaactc cacgaaaata tccgaacgca 4860
   gcaagatcgg tcgatcgact cagatctggg taactggcct aactggcctt ggaggagctg 4920
   gcaactcaaa atccctttgc caaaaaccaa catcatgcca tccaccatgc ttgtatccag 4980
   ccgcgcgcaa tgtaccccgc gctgtgtatc ccaaagcctc atgcaaccta acagatggat 5040
   cgtttggaag gcctataaca gcaaccacag acttaaaacc ttgcgcctcc atagacttaa 5100
   gcaaatgtgt gtacaatgta gatcctaggc ccaacctttg atgcctatgt gacacgtaaa 5160
   cagtactctc aactgtccaa tcgtaagcgt tcctagcctt ccagggccca gcgtaagcaa 5220
   taccagccac aacaccctca acctcagcaa ccaaccaagg gtatctatct tgcaacctct 5280
   ctaggtcatc aatccactct tgtggtgttt gtggctctgt cctaaagttc actgtagacg 5340
   tctcaatgta atggttaacg atgtcacaaa ccgcggccat atcggctgct gtagctggcc 5400
   taatctcaac tggtctcctc tccggagaca tgtcgagatt atttggattg agagtgaata 5460
   tgagactcta attggatacc gaggggaatt tatggaacgt cagtggagca tttttgacaa 5520
   gaaatatttg ctagctgata gtgaccttag gcgacttttg aacgcgcaat aatggtttct 5580
   gacgtatgtg cttagctcat taaactccag aaacccgcgg ctgagtggct ccttcaacgt 5640
   tgcggttctg tcagttccaa acgtaaaacg gcttgtcccg cgtcatcggc gggggtcata 5700
   acgtgactcc cttaattctc cgctcatgat cagattgtcg tttcccgcct tcagtttaaa 5760
   ctatcagtgt ttgacaggat cctgcttggt aataattgtc attagattgt ttttatgcat 5820
   agatgcactc gaaatcagcc aattttagac aagtatcaaa cggatgttaa ttcagtacat 5880
   taaagacgtc cgcaatgtgt tattaagttg tctaagcgtc aatttgttta caccacaata 5940
   tatcctgcca ccagccagcc aacagctccc cgaccggcag ctcggcacaa aatcaccacg 6000
   cgttaccacc acgccggccg gccgcatggt gttgaccgtg ttcgccggca ttgccgagtt 6060
   cgagcgttcc ctaatcatcg accgcacccg gagcgggcgc gaggccgcca aggcccgagg 6120
   cgtgaagttt ggcccccgcc ctaccctcac cccggcacag atcgcgcacg cccgcgagct 6180
   gatcgaccag gaaggccgca ccgtgaaaga ggcggctgca ctgcttggcg tgcatcgctc 6240
   gaccctgtac cgcgcacttg agcgcagcga ggaagtgacg cccaccgagg ccaggcggcg 6300
   cggtgccttc cgtgaggacg cattgaccga ggccgacgcc ctggcggccg ccgagaatga 6360
   acgccaagag gaacaagcat gaaaccgcac caggacggcc aggacgaacc gtttttcatt 6420
   accgaagaga tcgaggcgga gatgatcgcg gccgggtacg tgttcgagcc gcccgcgcac 6480
   gtctcaaccg tgcggctgca tgaaatcctg gccggtttgt ctgatgccaa gctggcggcc 6540
   tggccggcca gcttggccgc tgaagaaacc gagcgccgcc gtctaaaaag gtgatgtgta 6600
   tttgagtaaa acagcttgcg tcatgcggtc gctgcgtata tgatgcgatg agtaaataaa 6660
   caaatacgca aggggaacgc atgaaggtta tcgctgtact taaccagaaa ggcgggtcag 6720
   gcaagacgac catcgcaacc catctagccc gcgccctgca actcgccggg gccgatgttc 6780
   tgttagtcga ttccgatccc cagggcagtg cccgcgattg ggcggccgtg cgggaagatc 6840
   aaccgctaac cgttgtcggc atcgaccgcc cgacgattga ccgcgacgtg aaggccatcg 6900
   gccggcgcga cttcgtagtg atcgacggag cgccccaggc ggcggacttg gctgtgtccg 6960
   cgatcaaggc agccgacttc gtgctgattc cggtgcagcc aagcccttac gacatatggg 7020
   ccaccgccga cctggtggag ctggttaagc agcgcattga ggtcacggat ggaaggctac 7080
   aagcggcctt tgtcgtgtcg cgggcgatca aaggcacgcg catcggcggt gaggttgccg 7140
   aggcgctggc cgggtacgag ctgcccattc ttgagtcccg tatcacgcag cgcgtgagct 7200
   acccaggcac tgccgccgcc ggcacaaccg ttcttgaatc agaacccgag ggcgacgctg 7260
   cccgcgaggt ccaggcgctg gccgctgaaa ttaaatcaaa actcatttga gttaatgagg 7320
   taaagagaaa atgagcaaaa gcacaaacac gctaagtgcc ggccgtccga gcgcacgcag 7380
   cagcaaggct gcaacgttgg ccagcctggc agacacgcca gccatgaagc gggtcaactt 7440
   tcagttgccg gcggaggatc acaccaagct gaagatgtac gcggtacgcc aaggcaagac 7500
   cattaccgag ctgctatctg aatacatcgc gcagctacca gagtaaatga gcaaatgaat 7560
   aaatgagtag atgaatttta gcggctaaag gaggcggcat ggaaaatcaa gaacaaccag 7620
   gcaccgacgc cgtggaatgc cccatgtgtg gaggaacggg cggttggcca ggcgtaagcg 7680
   gctgggttgt ctgccggccc tgcaatggca ctggaacccc caagcccgag gaatcggcgt 7740
   gagcggtcgc aaaccatccg gcccggtaca aatcggcgcg gcgctgggtg atgacctggt 7800
   ggagaagttg aaggccgcgc aggccgccca gcggcaacgc atcgaggcag aagcacgccc 7860
   cggtgaatcg tggcaagcgg ccgctgatcg aatccgcaaa gaatcccggc aaccgccggc 7920
   agccggtgcg ccgtcgatta ggaagccgcc caagggcgac gagcaaccag attttttcgt 7980
   tccgatgctc tatgacgtgg gcacccgcga tagtcgcagc atcatggacg tggccgtttt 8040
   ccgtctgtcg aagcgtgacc gacgagctgg cgaggtgatc cgctacgagc ttccagacgg 8100
   gcacgtagag gtttccgcag ggccggccgg catggccagt gtgtgggatt acgacctggt 8160
   actgatggcg gtttcccatc taaccgaatc catgaaccga taccgggaag ggaagggaga 8220
   caagcccggc cgcgtgttcc gtccacacgt tgcggacgta ctcaagttct gccggcgagc 8280
   cgatggcgga aagcagaaag acgacctggt agaaacctgc attcggttaa acaccacgca 8340
   cgttgccatg cagcgtacga agaaggccaa gaacggccgc ctggtgacgg tatccgaggg 8400
   tgaagccttg attagccgct acaagatcgt aaagagcgaa accgggcggc cggagtacat 8460
   cgagatcgag ctagctgatt ggatgtaccg cgagatcaca gaaggcaaga acccggacgt 8520
   gctgacggtt caccccgatt actttttgat cgatcccggc atcggccgtt ttctctaccg 8580
   cctggcacgc cgcgccgcag gcaaggcaga agccagatgg ttgttcaaga cgatctacga 8640
   acgcagtggc agcgccggag agttcaagaa gttctgtttc accgtgcgca agctgatcgg 8700
   gtcaaatgac ctgccggagt acgatttgaa ggaggaggcg gggcaggctg gcccgatcct 8760
   agtcatgcgc taccgcaacc tgatcgaggg cgaagcatcc gccggttcct aatgtacgga 8820
   gcagatgcta gggcaaattg ccctagcagg ggaaaaaggt cgaaaaggtc tctttcctgt 8880
   ggatagcacg tacattggga acccaaagcc gtacattggg aaccggaacc cgtacattgg 8940
   gaacccaaag ccgtacattg ggaaccggtc acacatgtaa gtgactgata taaaagagaa 9000
   aaaaggcgat ttttccgcct aaaactcttt aaaacttatt aaaactctta aaacccgcct 9060
   ggcctgtgca taactgtctg gccagcgcac agccgaagag ctgcaaaaag cgcctaccct 9120
   tcggtcgctg cgctccctac gccccgccgc ttcgcgtcgg cctatcgcgg ccgctggccg 9180
   ctcaaaaatg gctggcctac ggccaggcaa tctaccaggg cgcggacaag ccgcgccgtc 9240
   gccactcgac cgccggcgcc cacatcaagg caccctgcct cgcgcgtttc ggtgatgacg 9300
   gtgaaaacct ctgacacatg cagctcccgg agacggtcac agcttgtctg taagcggatg 9360
   ccgggagcag acaagcccgt cagggcgcgt cagcgggtgt tggcgggtgt cggggcgcag 9420
   ccatgaccca gtcacgtagc gatagcggag tgtatactgg cttaactatg cggcatcaga 9480
   gcagattgta ctgagagtgc accatatgcg gtgtgaaata ccgcacagat gcgtaaggag 9540
   aaaataccgc atcaggcgct cttccgcttc ctcgctcact gactcgctgc gctcggtcgt 9600
   tcggctgcgg cgagcggtat cagctcactc aaaggcggta atacggttat ccacagaatc 9660
   aggggataac gcaggaaaga acatgtgagc aaaaggccag caaaaggcca ggaaccgtaa 9720
   aaaggccgcg ttgctggcgt ttttccatag gctccgcccc cctgacgagc atcacaaaaa 9780
   tcgacgctca agtcagaggt ggcgaaaccc gacaggacta taaagatacc aggcgtttcc 9840
   ccctggaagc tccctcgtgc gctctcctgt tccgaccctg ccgcttaccg gatacctgtc 9900
   cgcctttctc ccttcgggaa gcgtggcgct ttctcatagc tcacgctgta ggtatctcag 9960
   ttcggtgtag gtcgttcgct ccaagctggg ctgtgtgcac gaaccccccg ttcagcccga 10020
   ccgctgcgcc ttatccggta actatcgtct tgagtccaac ccggtaagac acgacttatc 10080
   gccactggca gcagccactg gtaacaggat tagcagagcg aggtatgtag gcggtgctac 10140
   agagttcttg aagtggtggc ctaactacgg ctacactaga aggacagtat ttggtatctg 10200
   cgctctgctg aagccagtta ccttcggaaa aagagttggt agctcttgat ccggcaaaca 10260
   aaccaccgct ggtagcggtg gtttttttgt ttgcaagcag cagattacgc gcagaaaaaa 10320
   aggatctcaa gaagatcctt tgatcttttc tacggggtct gacgctcagt ggaacgaaaa 10380
   ctcacgttaa gggattttgg tcatgcatga tatatctccc aatttgtgta gggcttatta 10440
   tgcacgctta aaaataataa aagcagactt gacctgatag tttggctgtg agcaattatg 10500
   tgcttagtgc atctaacgct tgagttaagc cgcgccgcga agcggcgtcg gcttgaacga 10560
   atttctagct agacattatt tgccgactac cttggtgatc tcgcctttca cgtagtggac 10620
   aaattcttcc aactgatctg cgcgcgaggc caagcgatct tcttcttgtc caagataagc 10680
   ctgtctagct tcaagtatga cgggctgata ctgggccggc aggcgctcca ttgcccagtc 10740
   ggcagcgaca tccttcggcg cgattttgcc ggttactgcg ctgtaccaaa tgcgggacaa 10800
   cgtaagcact acatttcgct catcgccagc ccagtcgggc ggcgagttcc atagcgttaa 10860
   ggtttcattt agcgcctcaa atagatcctg ttcaggaacc ggatcaaaga gttcctccgc 10920
   cgctggacct accaaggcaa cgctatgttc tcttgctttt gtcagcaaga tagccagatc 10980
   aatgtcgatc gtggctggct cgaagatacc tgcaagaatg tcattgcgct gccattctcc 11040
   aaattgcagt tcgcgcttag ctggataacg ccacggaatg atgtcgtcgt gcacaacaat 11100
   ggtgacttct acagcgcgga gaatctcgct ctctccaggg gaagccgaag tttccaaaag 11160
   gtcgttgatc aaagctcgcc gcgttgtttc atcaagcctt acggtcaccg taaccagcaa 11220
   atcaatatca ctgtgtggct tcaggccgcc atccactgcg gagccgtaca aatgtacggc 11280
   cagcaacgtc ggttcgagat ggcgctcgat gacgccaact acctctgata gttgagtcga 11340
   tacttcggcg atcaccgctt cccccatgat gtttaacttt gttttagggc gactgccctg 11400
   ctgcgtaaca tcgttgctgc tccataacat caaacatcga cccacggcgt aacgcgcttg 11460
   ctgcttggat gcccgaggca tagactgtac cccaaaaaaa cagtcataac aagccatgaa 11520
   aaccgccact gcg 11533
<210> 4
   <211> 11533
   <212> DNA
   <213> Artificial Sequence
<220>
   <223> Expression vector UH201
<400> 4
   ttccatggac atacaaatgg acgaacggat aaaccttttc acgccctttt aaatatccga 60
   ttattctaat aaacgctctt ttctcttagg tttacccgcc aatatatcct gtcaaacact 120
   gatagtttaa actgaaggcg ggaaacgaca atcagatcta gtaggaaaca gctatgacca 180
   tgattacgcc aagcttgcat gccgatcccc cccactccgc cctacactcg tatatatatg 240
   cctaaacctg ccccgttcct catatgtgat attattattt cattattagg tataagatag 300
   taaacgataa ggaaagacaa tttattgaga aagccatgct aaaatataga tagatatacc 360
   ttagcaggtg tttattttac aacataacat aacatagtag ctagccagca ggcaggctaa 420
   aacatagtat agtctatctg cagggggtac ggtcgactct agactagtgg atccgtcgaa 480
   gctagcttgg gtcccgctca gaagaactcg tcaagaaggc gatagaaggc gatgcgctgc 540
   gaatcgggag cggcgatacc gtaaagcacg aggaagcggt cagcccattc gccgccaagc 600
   tcttcagcaa tatcacgggt agccaacgct atgtcctgat agcggtccgc cacacccagc 660
   cggccacagt cgatgaatcc agaaaagcgg ccattttcca ccatgatatt cggcaagcag 720
   gcatcgccat gggtcacgac gagatcctcg ccgtcgggca tgcgcgcctt gagcctggcg 780
   aacagttcgg ctggcgcgag cccctgatgc tcttcgtcca gatcatcctg atcgacaaga 840
   ccggcttcca tccgagtacg tgctcgctcg atgcgatgtt tcgcttggtg gtcgaatggg 900
   caggtagccg gatcaagcgt atgcagccgc cgcattgcat cagccatgat ggatactttc 960
   tcggcaggag caaggtgaga tgacaggaga tcctgccccg gcacttcgcc caatagcagc 1020
   cagtcccttc ccgcttcagt gacaacgtcg agcacagctg cgcaaggaac gcccgtcgtg 1080
   gccagccacg atagccgcgc tgcctcgtcc tgcagttcat tcagggcacc ggacaggtcg 1140
   gtcttgacaa aaagaaccgg gcgcccctgc gctgacagcc ggaacacggc ggcatcagag 1200
   cagccgattg tctgttgtgc ccagtcatag ccgaatagcc tctccaccca agcggccgga 1260
   gaacctgcgt gcaatccatc ttgttcaatc caagctccca tgggccctcg actagagtcg 1320
   agatccgata tcgcccgggc tcgactctag aggatccaag cttcactgct taaattcaca 1380
   aaaagagaaa agtaagacca aaggaataaa tcatcctcaa accaaaaaca catcatacaa 1440
   aatcatcaaa cataaatctc cagatgtatg agcaccaatc cagttataca acactcttaa 1500
   caccaaatca acagatttaa cagcgaaata agcttaagcc catacaatta tccgatccaa 1560
   acaaatataa tcgaaaccgg cagaggaata agcaagtgaa tcaaaaagta tgggacgagg 1620
   aagaagatga tacctgaatg agaaagtcaa taaccttgac ccgaatcgtt ttgaagaaaa 1680
   tggagaaaat cggttgtatg gaataaaatc ttcgaatgat gagatatatg atctctttgg 1740
   tgtcagtcac atggcacacg ctatcaattt agaaaaacgc ggtggttggt caccagaatt 1800
   actacttctc ggtctgattt ggtcatatcc gtattaagtc cggttaatat tttccataac 1860
   tggggtttga acattcggtt tctttttttc agttagtccg atttggagtt ttgagtatgg 1920
   aaaaataata ctgaatttat ttgttcaaac tgttttggaa aaaatatttc ccttaattac 1980
   gaatataatt aaaattttaa aattcatttt attagatctt ggttaattcg gtttaatgca 2040
   ttaatgaatt tcggtttaag tcggttttcg gtttttatgt cccaccacta tctacaaccg 2100
   atgatcaacc ttatctccgt attcaccaca aacagtcatc actctcactt gacacaaaaa 2160
   ctcttttgtc tccgtctctc tgtctctcgg atccccgggt aggtcagtcc cttatgttac 2220
   gtcctgtaga aaccccaacc cgtgaaatca aaaaactcga cggcctgtgg gcattcagtc 2280
   tggatcgcga aaactgtgga attggtcagc gttggtggga aagcgcgtta caagaaagcc 2340
   gggcaattgc tgtgccagga gtttttaacg atcaagttcg ccgatgccag atattcgtaa 2400
   ttatgccggc aacgtcttgg tatcagcgcc gaagtcttta ttccgaaagg ttgggcaggc 2460
   cagcgtatcg tgctgcgttt cgatgcggtc actcattacg gcaaagtgtg ggtcaataat 2520
   caggaagtga tggagcatca gggcggctat acgccatttg aagccgatgt cacgccgtat 2580
   gttattgccg ggaaaagtgt acgtaagttt ctgcttctac ctttgatata tatataataa 2640
   ttatcattaa ttagtagtaa tataatattt caaatatttt tttcaaaata aaagaatgta 2700
   gtatatagca attgcttttc tgtagtttat aagtgtgtat attttaattt ataacttttc 2760
   taatatatga ccaaaatttg ttgatgtgca ggtatcaccg tttgtgtgaa caacgaactg 2820
   aactggcaga ctatcccgcc gggaatggtg attaccgacg aaaacggcaa gaaaaagcag 2880
   tcttacttcc atgatttctt taactatgcc ggaatccatc gcagcgtaat gctctacacc 2940
   acgccgaaca cctgggtgga cgatatcacc gtggtgacgc atgtcgcgca agactgtaac 3000
   cacgcgtctg ttgactggca ggtggtggcc aatggtgatg tcagcgttga actgcgtgat 3060
   gcggatcaac aggtggttgc aactggacaa ggcactagcg ggactttgca agtggtgaat 3120
   ccgcacctct ggcaaccggg tgaaggttat ctctatgaac tgtgcgtcac agccaaaagc 3180
   cagacagagt gtgatatcta cccgcttcgc gtcggcatcc ggtcagtggc agtgaagggc 3240
   gaacagttcc tgattaacca caaaccgttc tactttactg gctttggtcg tcatgaagat 3300
   gcggacttac gtggcaaagg attcgataac gtgctgatgg tgcacgacca cgcattaatg 3360
   gactggattg gggccaactc ctaccgtacc tcgcattacc cttacgctga agagatgctc 3420
   gactgggcag atgaacatgg catcgtggtg attgatgaaa ctgctgctgt cggctttaac 3480
   ctctctttag gcattggttt cgaagcgggc aacaagccga aagaactgta cagcgaagag 3540
   gcagtcaacg gggaaactca gcaagcgcac ttacaggcga ttaaagagct gatagcgcgt 3600
   gacaaaaacc acccaagcgt ggtgatgtgg agtattgcca acgaaccgga tacccgtccg 3660
   caagtgcacg ggaatatttc gccactggcg gaagcaacgc gtaaactcga cccgacgcgt 3720
   ccgatcacct gcgtcaatgt aatgttctgc gacgctcaca ccgataccat cagcgatctc 3780
   tttgatgtgc tgtgcctgaa ccgttattac ggatggtatg tccaaagcgg cgatttggaa 3840
   acggcagaga aggtactgga aaaagaactt ctggcctggc aggagaaact gcatcagccg 3900
   attatcatca ccgaatacgg cgtggatacg ttagccgggc tgcactcaat gtacaccgac 3960
   atgtggagtg aagagtatca gtgtgcatgg ctggatatgt atcaccgcgt ctttgatcgc 4020
   gtcagcgccg tcgtcggtga acaggtatgg aatttcgccg attttgcgac ctcgcaaggc 4080
   atattgcgcg ttggcggtaa caagaaaggg atcttcactc gcgaccgcaa accgaagtcg 4140
   gcggcttttc tgctgcaaaa acgctggact ggcatgaact tcggtgaaaa accgcagcag 4200
   ggaggcaaac aatgaatcaa caactctcct ggcgcaccat cgtcggctac agcctcggga 4260
   attgctaccg agctcggtac ccggcgcaaa aatcaccagt ctctctctac aaatctatct 4320
   ctctctattt ttctccagaa taatgtgtga gtagttccca gataagggaa ttagggttct 4380
   tatagggttt cgctcatgtg ttgagcatat aagaaaccct tagtatgtat ttgtatttgt 4440
   aaaatacttc tatcaataaa atttctaatt cctaaaacca aaatccagtg accgggtacc 4500
   gagctcgaat tcactggccg tcgttttaca acgactcagc agcttgacag gaggcccgat 4560
   ctagtaacat agatgacacc gcgcgcgata atttatccta gtttgcgcgc tatattttgt 4620
   tttctatcgc gtattaaatg tataattgcg ggactctaat cataaaaacc catctcataa 4680
   ataacgtcat gcattacatg ttaattatta catgcttaac gtaattcaac agaaattata 4740
   tgataatcat cgcaagaccg gcaacaggat tcaatcttaa gaaactttat tgccaaatgt 4800
   ttgaacgatc ggggatcatc cgggtctgtg gcgggaactc cacgaaaata tccgaacgca 4860
   gcaagatcgg tcgatcgact cagatctggg taactggcct aactggcctt ggaggagctg 4920
   gcaactcaaa atccctttgc caaaaaccaa catcatgcca tccaccatgc ttgtatccag 4980
   ccgcgcgcaa tgtaccccgc gctgtgtatc ccaaagcctc atgcaaccta acagatggat 5040
   cgtttggaag gcctataaca gcaaccacag acttaaaacc ttgcgcctcc atagacttaa 5100
   gcaaatgtgt gtacaatgta gatcctaggc ccaacctttg atgcctatgt gacacgtaaa 5160
   cagtactctc aactgtccaa tcgtaagcgt tcctagcctt ccagggccca gcgtaagcaa 5220
   taccagccac aacaccctca acctcagcaa ccaaccaagg gtatctatct tgcaacctct 5280
   ctaggtcatc aatccactct tgtggtgttt gtggctctgt cctaaagttc actgtagacg 5340
   tctcaatgta atggttaacg atgtcacaaa ccgcggccat atcggctgct gtagctggcc 5400
   taatctcaac tggtctcctc tccggagaca tgtcgagatt atttggattg agagtgaata 5460
   tgagactcta attggatacc gaggggaatt tatggaacgt cagtggagca tttttgacaa 5520
   gaaatatttg ctagctgata gtgaccttag gcgacttttg aacgcgcaat aatggtttct 5580
   gacgtatgtg cttagctcat taaactccag aaacccgcgg ctgagtggct ccttcaacgt 5640
   tgcggttctg tcagttccaa acgtaaaacg gcttgtcccg cgtcatcggc gggggtcata 5700
   acgtgactcc cttaattctc cgctcatgat cagattgtcg tttcccgcct tcagtttaaa 5760
   ctatcagtgt ttgacaggat cctgcttggt aataattgtc attagattgt ttttatgcat 5820
   agatgcactc gaaatcagcc aattttagac aagtatcaaa cggatgttaa ttcagtacat 5880
   taaagacgtc cgcaatgtgt tattaagttg tctaagcgtc aatttgttta caccacaata 5940
   tatcctgcca ccagccagcc aacagctccc cgaccggcag ctcggcacaa aatcaccacg 6000
   cgttaccacc acgccggccg gccgcatggt gttgaccgtg ttcgccggca ttgccgagtt 6060
   cgagcgttcc ctaatcatcg accgcacccg gagcgggcgc gaggccgcca aggcccgagg 6120
   cgtgaagttt ggcccccgcc ctaccctcac cccggcacag atcgcgcacg cccgcgagct 6180
   gatcgaccag gaaggccgca ccgtgaaaga ggcggctgca ctgcttggcg tgcatcgctc 6240
   gaccctgtac cgcgcacttg agcgcagcga ggaagtgacg cccaccgagg ccaggcggcg 6300
   cggtgccttc cgtgaggacg cattgaccga ggccgacgcc ctggcggccg ccgagaatga 6360
   acgccaagag gaacaagcat gaaaccgcac caggacggcc aggacgaacc gtttttcatt 6420
   accgaagaga tcgaggcgga gatgatcgcg gccgggtacg tgttcgagcc gcccgcgcac 6480
   gtctcaaccg tgcggctgca tgaaatcctg gccggtttgt ctgatgccaa gctggcggcc 6540
   tggccggcca gcttggccgc tgaagaaacc gagcgccgcc gtctaaaaag gtgatgtgta 6600
   tttgagtaaa acagcttgcg tcatgcggtc gctgcgtata tgatgcgatg agtaaataaa 6660
   caaatacgca aggggaacgc atgaaggtta tcgctgtact taaccagaaa ggcgggtcag 6720
   gcaagacgac catcgcaacc catctagccc gcgccctgca actcgccggg gccgatgttc 6780
   tgttagtcga ttccgatccc cagggcagtg cccgcgattg ggcggccgtg cgggaagatc 6840
   aaccgctaac cgttgtcggc atcgaccgcc cgacgattga ccgcgacgtg aaggccatcg 6900
   gccggcgcga cttcgtagtg atcgacggag cgccccaggc ggcggacttg gctgtgtccg 6960
   cgatcaaggc agccgacttc gtgctgattc cggtgcagcc aagcccttac gacatatggg 7020
   ccaccgccga cctggtggag ctggttaagc agcgcattga ggtcacggat ggaaggctac 7080
   aagcggcctt tgtcgtgtcg cgggcgatca aaggcacgcg catcggcggt gaggttgccg 7140
   aggcgctggc cgggtacgag ctgcccattc ttgagtcccg tatcacgcag cgcgtgagct 7200
   acccaggcac tgccgccgcc ggcacaaccg ttcttgaatc agaacccgag ggcgacgctg 7260
   cccgcgaggt ccaggcgctg gccgctgaaa ttaaatcaaa actcatttga gttaatgagg 7320
   taaagagaaa atgagcaaaa gcacaaacac gctaagtgcc ggccgtccga gcgcacgcag 7380
   cagcaaggct gcaacgttgg ccagcctggc agacacgcca gccatgaagc gggtcaactt 7440
   tcagttgccg gcggaggatc acaccaagct gaagatgtac gcggtacgcc aaggcaagac 7500
   cattaccgag ctgctatctg aatacatcgc gcagctacca gagtaaatga gcaaatgaat 7560
   aaatgagtag atgaatttta gcggctaaag gaggcggcat ggaaaatcaa gaacaaccag 7620
   gcaccgacgc cgtggaatgc cccatgtgtg gaggaacggg cggttggcca ggcgtaagcg 7680
   gctgggttgt ctgccggccc tgcaatggca ctggaacccc caagcccgag gaatcggcgt 7740
   gagcggtcgc aaaccatccg gcccggtaca aatcggcgcg gcgctgggtg atgacctggt 7800
   ggagaagttg aaggccgcgc aggccgccca gcggcaacgc atcgaggcag aagcacgccc 7860
   cggtgaatcg tggcaagcgg ccgctgatcg aatccgcaaa gaatcccggc aaccgccggc 7920
   agccggtgcg ccgtcgatta ggaagccgcc caagggcgac gagcaaccag attttttcgt 7980
   tccgatgctc tatgacgtgg gcacccgcga tagtcgcagc atcatggacg tggccgtttt 8040
   ccgtctgtcg aagcgtgacc gacgagctgg cgaggtgatc cgctacgagc ttccagacgg 8100
   gcacgtagag gtttccgcag ggccggccgg catggccagt gtgtgggatt acgacctggt 8160
   actgatggcg gtttcccatc taaccgaatc catgaaccga taccgggaag ggaagggaga 8220
   caagcccggc cgcgtgttcc gtccacacgt tgcggacgta ctcaagttct gccggcgagc 8280
   cgatggcgga aagcagaaag acgacctggt agaaacctgc attcggttaa acaccacgca 8340
   cgttgccatg cagcgtacga agaaggccaa gaacggccgc ctggtgacgg tatccgaggg 8400
   tgaagccttg attagccgct acaagatcgt aaagagcgaa accgggcggc cggagtacat 8460
   cgagatcgag ctagctgatt ggatgtaccg cgagatcaca gaaggcaaga acccggacgt 8520
   gctgacggtt caccccgatt actttttgat cgatcccggc atcggccgtt ttctctaccg 8580
   cctggcacgc cgcgccgcag gcaaggcaga agccagatgg ttgttcaaga cgatctacga 8640
   acgcagtggc agcgccggag agttcaagaa gttctgtttc accgtgcgca agctgatcgg 8700
   gtcaaatgac ctgccggagt acgatttgaa ggaggaggcg gggcaggctg gcccgatcct 8760
   agtcatgcgc taccgcaacc tgatcgaggg cgaagcatcc gccggttcct aatgtacgga 8820
   gcagatgcta gggcaaattg ccctagcagg ggaaaaaggt cgaaaaggtc tctttcctgt 8880
   ggatagcacg tacattggga acccaaagcc gtacattggg aaccggaacc cgtacattgg 8940
   gaacccaaag ccgtacattg ggaaccggtc acacatgtaa gtgactgata taaaagagaa 9000
   aaaaggcgat ttttccgcct aaaactcttt aaaacttatt aaaactctta aaacccgcct 9060
   ggcctgtgca taactgtctg gccagcgcac agccgaagag ctgcaaaaag cgcctaccct 9120
   tcggtcgctg cgctccctac gccccgccgc ttcgcgtcgg cctatcgcgg ccgctggccg 9180
   ctcaaaaatg gctggcctac ggccaggcaa tctaccaggg cgcggacaag ccgcgccgtc 9240
   gccactcgac cgccggcgcc cacatcaagg caccctgcct cgcgcgtttc ggtgatgacg 9300
   gtgaaaacct ctgacacatg cagctcccgg agacggtcac agcttgtctg taagcggatg 9360
   ccgggagcag acaagcccgt cagggcgcgt cagcgggtgt tggcgggtgt cggggcgcag 9420
   ccatgaccca gtcacgtagc gatagcggag tgtatactgg cttaactatg cggcatcaga 9480
   gcagattgta ctgagagtgc accatatgcg gtgtgaaata ccgcacagat gcgtaaggag 9540
   aaaataccgc atcaggcgct cttccgcttc ctcgctcact gactcgctgc gctcggtcgt 9600
   tcggctgcgg cgagcggtat cagctcactc aaaggcggta atacggttat ccacagaatc 9660
   aggggataac gcaggaaaga acatgtgagc aaaaggccag caaaaggcca ggaaccgtaa 9720
   aaaggccgcg ttgctggcgt ttttccatag gctccgcccc cctgacgagc atcacaaaaa 9780
   tcgacgctca agtcagaggt ggcgaaaccc gacaggacta taaagatacc aggcgtttcc 9840
   ccctggaagc tccctcgtgc gctctcctgt tccgaccctg ccgcttaccg gatacctgtc 9900
   cgcctttctc ccttcgggaa gcgtggcgct ttctcatagc tcacgctgta ggtatctcag 9960
   ttcggtgtag gtcgttcgct ccaagctggg ctgtgtgcac gaaccccccg ttcagcccga 10020
   ccgctgcgcc ttatccggta actatcgtct tgagtccaac ccggtaagac acgacttatc 10080
   gccactggca gcagccactg gtaacaggat tagcagagcg aggtatgtag gcggtgctac 10140
   agagttcttg aagtggtggc ctaactacgg ctacactaga aggacagtat ttggtatctg 10200
   cgctctgctg aagccagtta ccttcggaaa aagagttggt agctcttgat ccggcaaaca 10260
   aaccaccgct ggtagcggtg gtttttttgt ttgcaagcag cagattacgc gcagaaaaaa 10320
   aggatctcaa gaagatcctt tgatcttttc tacggggtct gacgctcagt ggaacgaaaa 10380
   ctcacgttaa gggattttgg tcatgcatga tatatctccc aatttgtgta gggcttatta 10440
   tgcacgctta aaaataataa aagcagactt gacctgatag tttggctgtg agcaattatg 10500
   tgcttagtgc atctaacgct tgagttaagc cgcgccgcga agcggcgtcg gcttgaacga 10560
   atttctagct agacattatt tgccgactac cttggtgatc tcgcctttca cgtagtggac 10620
   aaattcttcc aactgatctg cgcgcgaggc caagcgatct tcttcttgtc caagataagc 10680
   ctgtctagct tcaagtatga cgggctgata ctgggccggc aggcgctcca ttgcccagtc 10740
   ggcagcgaca tccttcggcg cgattttgcc ggttactgcg ctgtaccaaa tgcgggacaa 10800
   cgtaagcact acatttcgct catcgccagc ccagtcgggc ggcgagttcc atagcgttaa 10860
   ggtttcattt agcgcctcaa atagatcctg ttcaggaacc ggatcaaaga gttcctccgc 10920
   cgctggacct accaaggcaa cgctatgttc tcttgctttt gtcagcaaga tagccagatc 10980
   aatgtcgatc gtggctggct cgaagatacc tgcaagaatg tcattgcgct gccattctcc 11040
   aaattgcagt tcgcgcttag ctggataacg ccacggaatg atgtcgtcgt gcacaacaat 11100
   ggtgacttct acagcgcgga gaatctcgct ctctccaggg gaagccgaag tttccaaaag 11160
   gtcgttgatc aaagctcgcc gcgttgtttc atcaagcctt acggtcaccg taaccagcaa 11220
   atcaatatca ctgtgtggct tcaggccgcc atccactgcg gagccgtaca aatgtacggc 11280
   cagcaacgtc ggttcgagat ggcgctcgat gacgccaact acctctgata gttgagtcga 11340
   tacttcggcg atcaccgctt cccccatgat gtttaacttt gttttagggc gactgccctg 11400
   ctgcgtaaca tcgttgctgc tccataacat caaacatcga cccacggcgt aacgcgcttg 11460
   ctgcttggat gcccgaggca tagactgtac cccaaaaaaa cagtcataac aagccatgaa 11520
   aaccgccact gcg 11533
<210> 5
   <211> 33
   <212> DNA
   <213> Artificial Sequence
<220>
   <223> Oligonucleotide primer
<400> 5
   acggatccga gagacagaga gacggagaca aaa 33
<210> 6
   <211> 28
   <212> DNA
   <213> Artificial Sequence
<220>
   <223> Oligonucleotide primer
<400> 6
   gcggatccaa gcttcactgc ttaaattc 28